(19) Europäisches Patentamt
European Patent Office
Office européen des brevets

(11) **EP 1 430 124 B1**

(12) **EUROPEAN PATENT SPECIFICATION**

(45) Date of publication and mention
of the grant of the patent:
**06.11.2013 Bulletin 2013/45**

(51) Int Cl.:
***C12N 15/11*** *(2006.01)*

(21) Application number: **01969944.6**

(22) Date of filing: **20.09.2001**

(86) International application number:
**PCT/GB2001/004210**

(87) International publication number:
**WO 2002/024930 (28.03.2002 Gazette 2002/12)**

(54) **ARTIFICIEL UBIQUITOUS CHROMATIN OPENING ELEMENTS (UCOE)**

ARTIFIZIELLE UBIQUITÄRE CHROMATIN ÖFFNUNGSELEMENTE (UCOE)

ELEMENTS ARTIFICIELS UBIQUISTES D'OUVERTURE DE LA CHROMATINE (UCOE)

(84) Designated Contracting States:
**AT BE CH CY DE DK ES FI FR GB GR IE IT LI LU MC NL PT SE TR**

(30) Priority: **20.09.2000 GB 0022995**
**20.11.2000 US 252048 P**

(43) Date of publication of application:
**23.06.2004 Bulletin 2004/26**

(60) Divisional application:
**10184573.3 / 2 365 076**

(73) Proprietor: **EMD Millipore Corporation**
**Billerica, MA 01821 (US)**

(72) Inventors:
• **ANTONIOU, Michael**
**Keele,**
**staffordshire ST5 5SP (GB)**
• **CROMBIE, Robert**
**Keele,**
**staffordshire ST5 5SP (GB)**
• **WILLIAMS, Steve, Geraint**
**Crewe**
**Cheshire CW2 5RE (GB)**

(74) Representative: **Ward, Siobhan et al**
**Harrison Goddard Foote LLP**
**8th Floor**
**140 London Wall**
**London EC2Y 5DN (GB)**

(56) References cited:
**WO-A-00/05393**

• **TAZI J. AND BIRD A.: "Alternative Chromatin Structure at CpG Islands", CELL, vol. 60, 1990, - 1990, pages 909-920,**
• **KUNDU T. K. AND RAO M. R. S.: "CpC Islands in Chromatin Organization and Gene Expression", J. BIOCHEM., vol. 125, no. 2, 1999, pages 217-222,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to a polynucleotide comprising a ubiquitous chromatin opening element (UCOE) that does not occur in nature. The present invention also relates to a vector comprising the polynucleotide sequence, a host cell comprising the vector and use of the polynucleotide, vector or host cell in therapy, or for in vitro protein expression applications.

**BACKGROUND OF THE INVENTION**

The current model of chromatin structure in higher eukaryotes postulates that genes are organised in "domains"[1,2]. Chromatin domains are envisaged to exist in either a condensed, "closed", transcriptionally silent state, or in a de-condensed, "open" and transcriptionally competent configuration. The establishment of an open chromatin structure characterised by increased DNaseI sensitivity, DNA hypomethylation and histone hyperacetylation, is considered a prerequisite to the commencement of gene expression.

The open and closed nature of chromatin regions is reflected in the behaviour of transgenes that are randomly integrated into the host cell genome. Identical constructs give different patterns of tissue-specific and development stage-specific expression when integrated at different locations in the mouse genome[3-5]. A variegated expression pattern within a given transgenic mouse tissue, known as position effect variegation (PEV), is also frequently observed[6]. When exogenous genes are integrated into the chromosome of mammalian cells cultures *in vitro*, many of the integration events result in rapid silencing of the transgene and the remainder give large variability in expression levels[7,8]. These position effects render transgene expression inefficient, with implication for both basic research and biotechnology applications.

The chromatin domain model of gene organisation suggests that genetic control elements that are able to establish and maintain a transcriptionally competent open chromatin structure should be associated with active regions of the genome.

Locus Control Regions (LCRs) are a class of transcriptional regulatory elements with long-range chromatin remodelling capability. LCRs are functionally defined in transgenic mice by their ability to confer site-of-integration independent, transgene copy number-dependent, physiological levels of expression on.a gene linked in *cis,* especially single copy transgenes[9,10]. Crucially, such expression is tissue-specific. LCRs are able to obstruct the spread of heterochromatin, prevent PEV[6] and consist of a series of Dnase I hypersensitive (HS) sites which can be located either 5' or 3' of the genes that they regulate[10].

LCRs appear to be comprised of two separate, although not necessary independent components. First, the establishment of an open chromatin domain, and second a dominant transcriptional activation capacity to confer transgene copy number dependent expression[9]. The molecular mechanisms by which LCRs exert their function remain a point of contention[2,11-13].

The generation of cultured mammalian cell lines producing high levels of a therapeutic protein product is a major developing industry. Chromatin position effects make it a difficult, time consuming and expensive process. The most commonly used approach to the production of such mammalian "cell factories" relies on gene amplification induced by a combination of a drug resistance gene (e.g., DHFR, glutamine synthetase (Kaufman, 1990, Methods Enzymol., 185:537-566)) and the maintenance of stringent selective pressure. The use of vectors containing LCRs from highlyexpressed gene domains, using cells derived from the appropriate tissue, greatly simplifies the procedure (Needham et al., 1992, Nucleic Acids Res., 20:997-1003; Needham et al., 1995, Protein Expr. Purif., 6:124-131).

However, the tissue-specificity of LCRs, although useful in some circumstances, is also a major limitation for many applications, for instance where no LCR is known for the tissue in which expression is required, or where expression in many, or all, tissues is required.

Our co-pending patent application PCT/GB99/02357 (WO 00/05393), describes elements that are responsible for establishing an open chromatin structure across a locus that consists exclusively of ubiquitously expressed, housekeeping genes. These elements are not derived from an LCR. The invention provides a polynucleotide comprising a ubiquitous chromatin opening element (UCOE) which opens chromatin or maintains chromatin in an open state and facilitates reproducible expression of an operably-linked gene in cells of at least two different tissue types, wherein the polynucleotide is not derived from a locus control region.

Methylation-free CpG islands are well-known in the art (Bird et al (1985) Cell 40: 91-99, Bird A.P (1986) Nature: 321:209-213, Tazi and Bird (1990) Cell 60: 909-920) and may be defined as CpG-rich regions of DNA with above average (>60%) content of CpG di-nucleotides where the cytosine residues are not methylated and which extend over the 5' ends of two closely spaced (0.1-3 kb) divergently transcribed genes. These regions of DNA remain unmethylated in all tissues throughout development (Wise and Pravtcheva (1999) Genomics 60: 258-271). They are associated with the 5' ends of all ubiquitously expressed genes, as well as an estimated 40% of genes showing a tissue restricted expression profile[16,17] and are known to be localised regions of active chromatin[18]. Kundu and Rao (1999) J. Biochem., 125:217-222 discloses that CpG islands are associated with all house-keeping genes and some tissue specific genes.

An 'extended' methylation-free CpG island is a methylation-free CpG island that extends across a region encompassing more than one transcriptional start site and/or extends for more than 300bp and preferably more than 500bp. The borders of the extended methylation-free CpG island are functionally defined through the use of PCR over the region in combination with restriction endonuclease enzymes whose ability to digest (cut) DNA at their recognition sequence is sensitive to the methylation status of any CpG residues that are present. One such enzyme is HpaII, which recognises and digests at the site CCGG, which is commonly found within CpG islands, but only if the central CG residues are *not* methylated. Therefore, PCR conducted with HpaII-digested DNA and over a region harbouring HpaII sites, does *not* give an amplification product due to HpaII digestion if the DNA is *unmethylated*. The PCR will *only* give an amplified product if the DNA is *methylated.* Therefore, beyond the methylation-free region HpaII will not digest the DNA a PCR amplified product will be observed thereby defining the boundaries of the "extended methylation-free CpG island".

We have demonstrated that regions spanning methylation-free CpG islands encompassing dual, divergently transcribed promoters from the human TATA binding protein (*TBP*)/proteosome component-B1 (*PSMBI*) and heterogenous nuclear ribonucleoprotein A2/B1 (*hnRNPA2*)/heterochromatin protein lHsy (*HP1Hsy*) gene loci give reproducible, physiological levels of gene expression and that they are able to prevent a variegated expression pattern and silencing that normally occurs with transgene integration within centromeric heterochomatin.

We have shown that methylation-free CpG islands associated with actively transcribing promoters possess the ability to remodel chromatin and are thus thought to be a prime determinant in establishing and-maintaining an open domain at housekeeping gene loci.

UCOE's confer an increased proportion of productive gene delivery events with improvements in the level and stability of transgene expression. This has important research and biotechnological applications including the generation of non-human transgenic animals and recombinant protein products in cultured cells. We have shown beneficial effects of UCOEs on expression of the CMV-EGFP reporter construct and with the secreted, pharmaceutically valuable protein erythropoietin. The properties of UCOEs also suggest utility in gene therapy, the effectiveness of which is often limited by a low frequency of productive gene delivery events and an inadequate level and duration of expression[45].

Given these significant implications and wide ranging applications, there is a desire to further optimise transgene expression levels and achieve improved stability of gene expression over a prolonged period of culture.

One particular need is to overcome the directional bias observed in some naturally-occurring UCOEs. Although UCOEs confer position-independent transcriptional enhancement on operably-linked promoters, this is, to some extent, orientation-dependent (i. e. the UCOE is significantly more effective in one orientation than the other). In some circumstances, such as an expression vector comprising two expression units transcribed divergently with a UCOE situated between them, there is an advantage in being able to obtain balanced, high-level expression from both promoters, which may not be possible with a natural UCOE. There is therefore a need for artificially-constructed UCOEs that are effective in both orientations.

STATEMENTS OF THE INVENTION

The present invention provides a method for aiding balanced expression of two divergently transcribed expression units according to any of claims 1 to 6.

The invention also provides a chimeric polypeptide according to any of claims 7 to 10 and a vector according to any of claims 11 to 13. The invention also provides a host cell according to claims 14 and 15.

In another aspect the invention provides the methods of claims 16 and 17 and a polynucleotide according to claim 18.

Also described is a polynucleotide comprising a UCOE, which opens chromatin or maintains chromatin in an open state and facilitates reproducible expression of an operably-linked gene in cells of at least two different tissue types, wherein the nucleotide sequence of UCOE does not occur in nature.

Genomic regions comprising regulatory sequences from at least two genes were combined to form chimeric UCOE which significantly enhanced gene expression over a prolonged period of culture. Such a chimeric UCOE constitutes a nucleotide sequence that does not occur in nature. Accordingly the phase "does not occur in nature" refers to a situation wherein the nucleotide sequence of the element constituting the UCOE does not naturally exist as such and is man-made or artificially constructed, being a combination of naturally-occurring and/or artificially -generated sequences.

As used herein, the terms "artificial", "artificially-constructed", "chimeric", and the like, in reference to a UCOE, are used interchangeably throughout to mean that the UCOE or the element constituting the UCOE does not naturally exist; i.e., "does not occur in nature." Where the UCOE is a combination of naturally-occurring sequences, it is their arrangement or organization into the UCOE that is non-natural. By way of non-limiting example, an artificial UCOE could be comprised of two naturally-occurring sequences that are normally disparate (from different regions of a chromosome, from different chromosomes, from different organisms, etc.) and that have been brought together in a non-natural organization, to create a chimeric or artificial UCOE.

As used herein, the terms "artificially synthesized" and "artificially-generated" in reference to sequences in a UCOE, refer to sequences that are non-natural; i.e., sequences that are not naturally-occurring and are wholly synthetic. Such "artificially synthesized" and "artificially-generated" sequences can also be combined with naturally-occurring sequences to make up or create an artificial UCOE.

Also described is a nucleic acid molecule comprising a DNA sequence selected from:

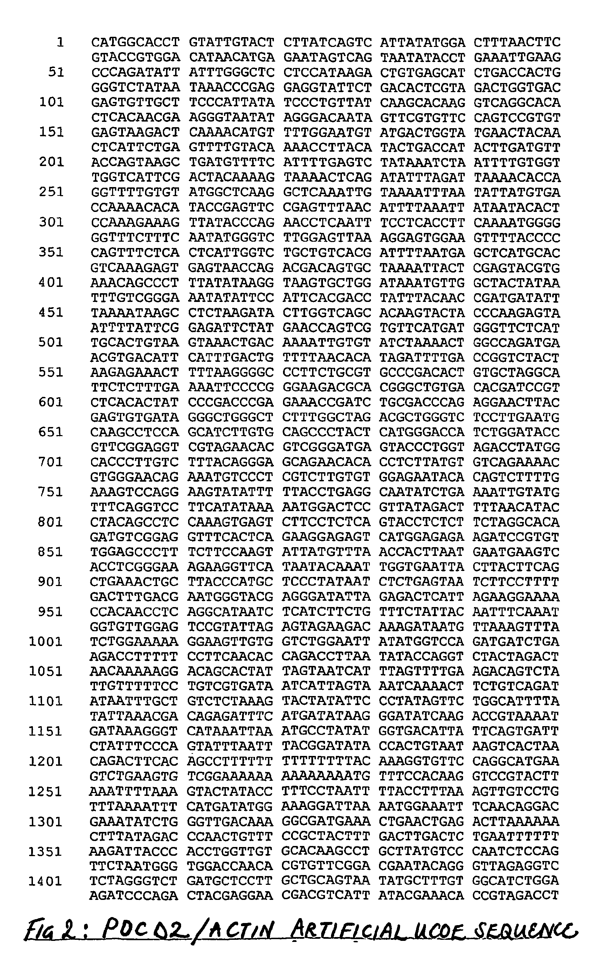

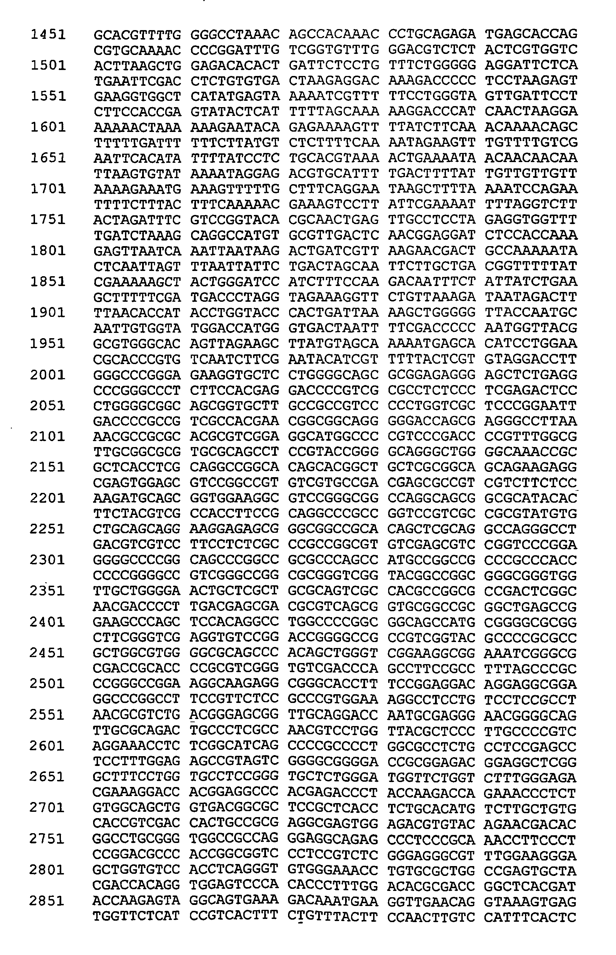

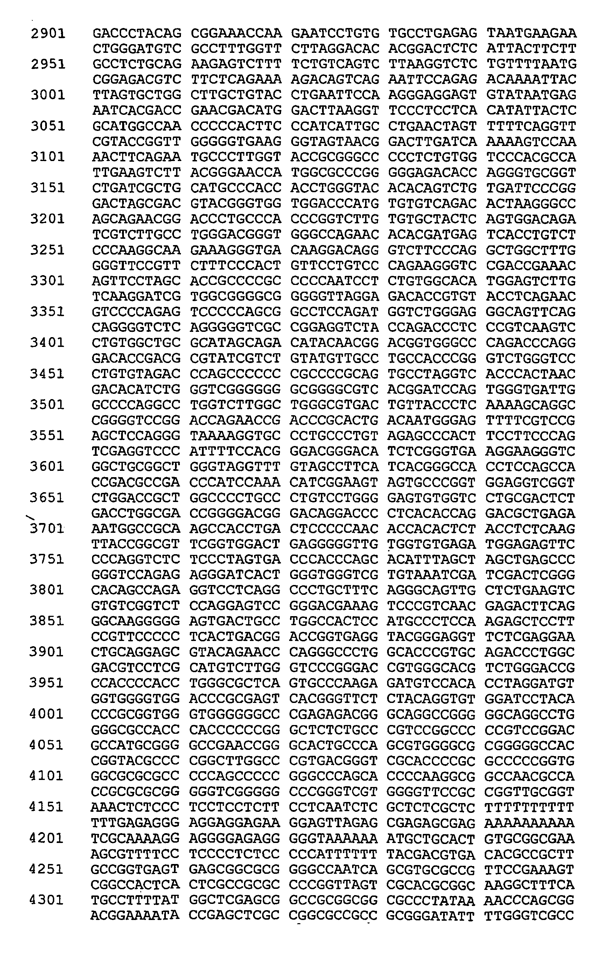

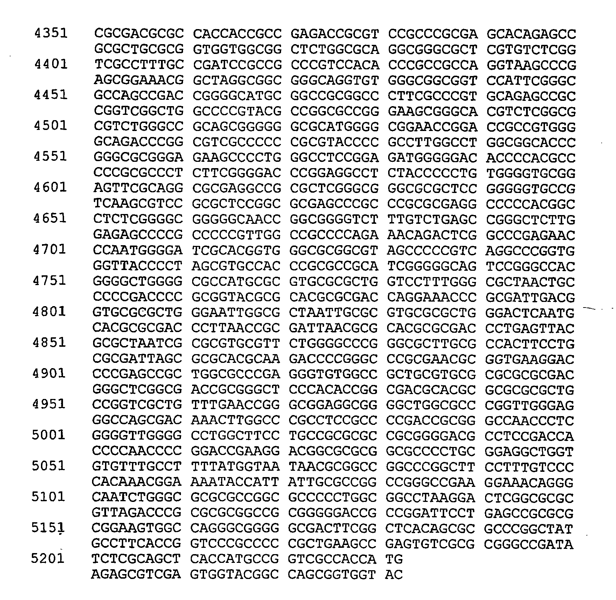

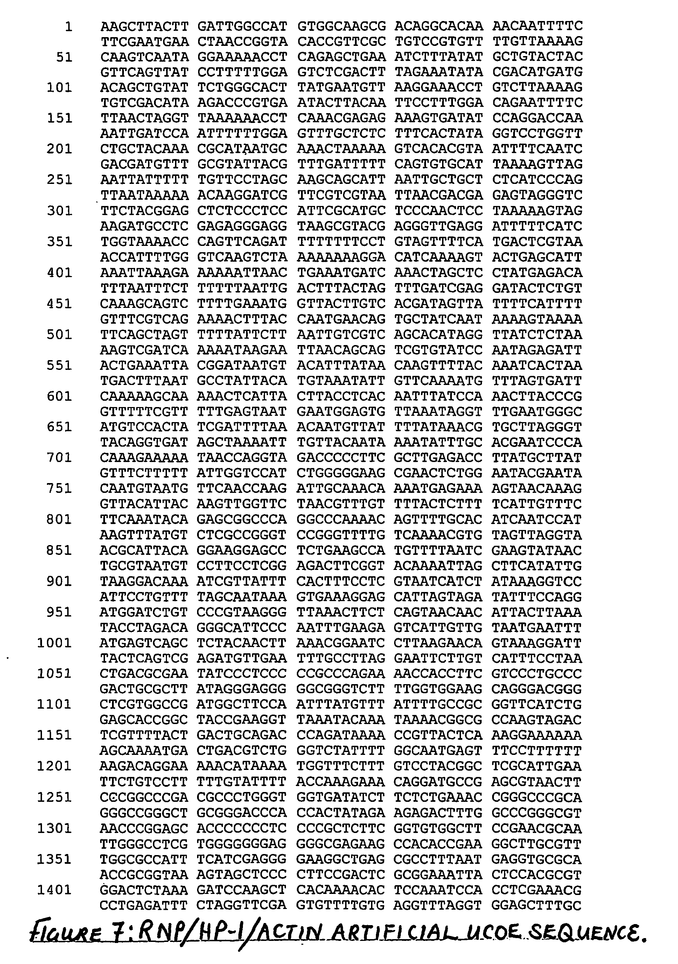

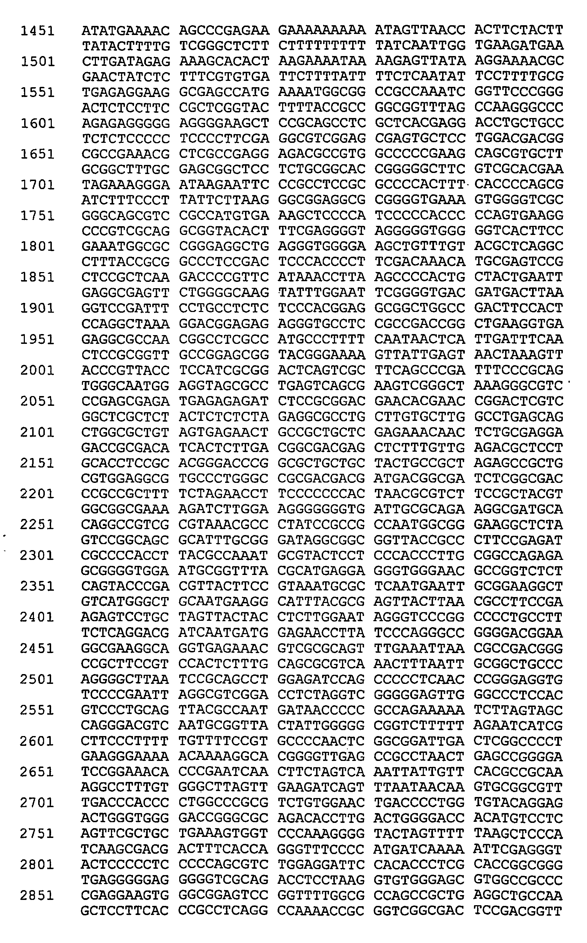

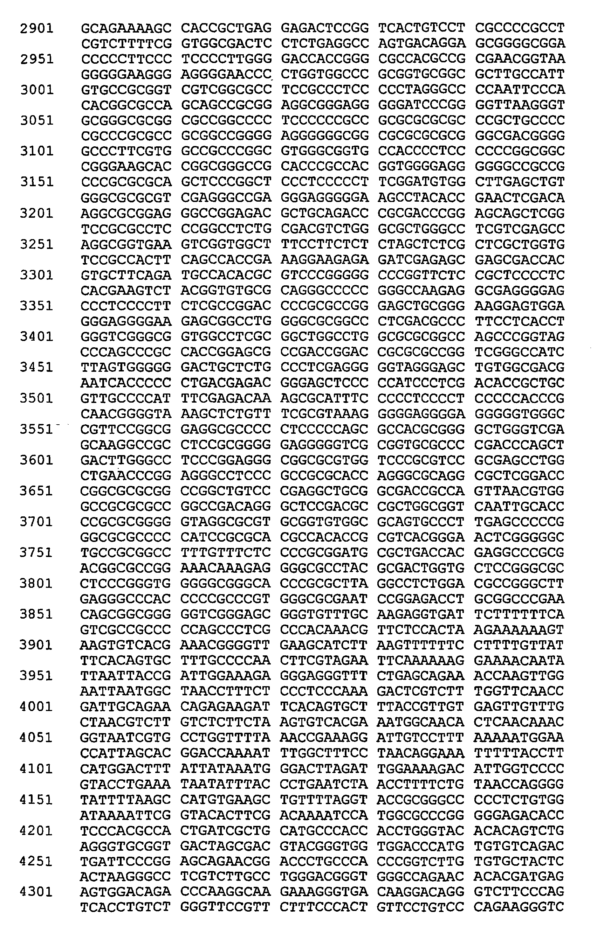

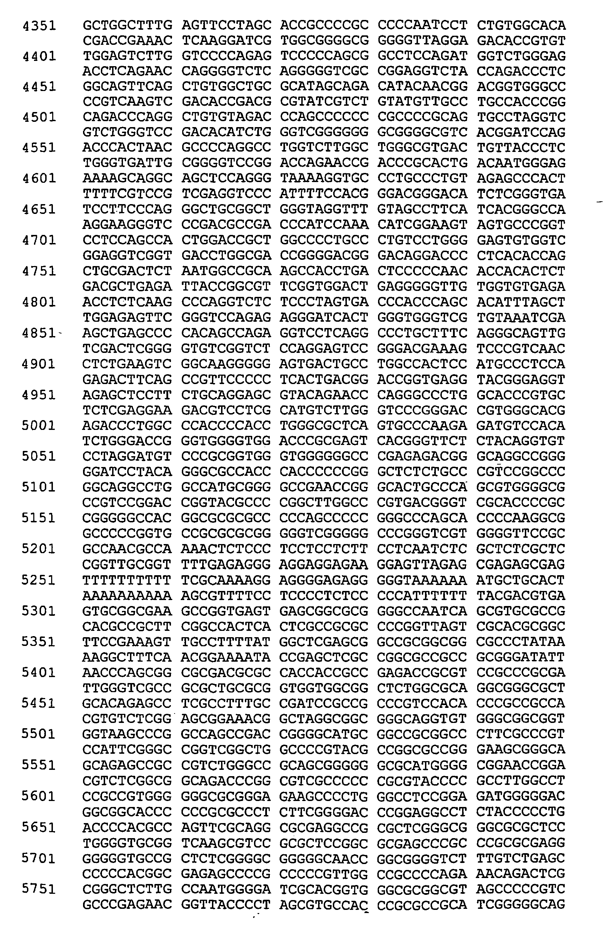

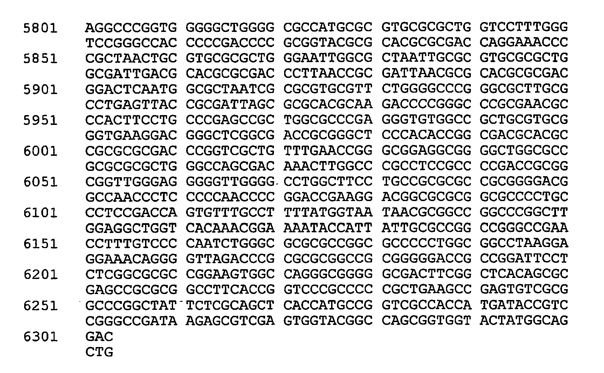

i) the DNA sequence as represented in Figure 2 or Figure 7;
ii) DNA sequences which hybridise to the sequence presented in Figure 2 or Figure 7 which encode a polypeptide according to the invention.

An isolated nucleic acid molecule which anneals under stringent hybridisation conditions to the sequence presented in Figure 2 or Figure 7 is also described.

Stringent hybridisation/washing conditions are well known in the art. For example, nucleic acid hybrids that are stable after washing in 0.1 x SSC, 0.1% SDS at 60°C. It is well known in the art that optimal hybridisation conditions can be calculated if the sequence of the nucleic acid is known. For example, hybridisation conditions can be determined by the GC content of the nucleic acid subject to hybridisation. Please see Sambrook et al (1989), Molecular Cloning; A Laboratory Approach. A common formula for calculating the stringency conditions required to achieve hybridisation between nucleic acid molecules of a specified homology is:

$$T_m = 81.5^0\,C + 16.6\,\text{Log}\,[Na^+] + 0.41[\,\%\,G + C] - 0.63\,(\%\text{formamide})$$

Preferably the polynucleotide facilitates reproducible expression of an operably-linked gene non-tissue specifically.

Preferably the polynucleotide facilitates reproducible expression of an operably-linked gene in all tissue types where active gene expression occurs.

Preferably the polynucleotide facilitates expression of an operably-linked gene at a physiological level.

Preferably the polynucleotide comprises an extended methylation-free, CpG-island.

Preferably the polynucleotide comprises one or more naturally-occuring sequences associated with the control of gene expression.

Preferably the polynucleotide comprises one or more naturally-occurring promoters.

Preferably the polynucleotide comprises dual or bidirectional promoters that transcribe divergently.

Preferably the polynucleotide comprises the human β-actin CpG island/promoter region, or fragment thereof.

Preferably the polynucleotide comprises a DNA fragment within the range of 100bp to 3 kb spanning the human β-actin CpG island/promoter region or a fragment thereof.

Preferably the polynucleotide comprises the human PDCD2 CpG island/promoter region or a fragment thereof.

Preferably the polynucleotide comprises a DNA fragment within the range from 100bp to 3.0 kb spanning the human PDCD2 CpG island/ promoter region, or a fragment thereof.

Preferably the polynucleotide comprises a DNA fragment within the range from 100bp to 3.0kb spanning the human β-actin CpG island/ promoter region and a DNA fragment within the range from 100bp to 3.0kb spanning the human PDCD2 CpG island/promoter region. Preferably said fragments are directly adjacent with their promoters oriented divergently.

Preferably the polynucleotide comprises a 2kb DNA fragment spanning the human β-actin CpG island/promoter region and a 1.8kb DNA fragment spanning the human PDCD2 CpG island/promoter region. Preferably said fragments are directly adjacent with their promoters oriented divergently.

As described the polynucleotide may comprise the sequence of Figure 2 or a fragment thereof, in either orientation.

Preferably the polynucleotide comprises the human RNP CpG island/promoter region or a fragment thereof.

Preferably the polynucleotide comprises a 4kb DNA fragment spanning the human RNP CpG island/promoter region.

Preferably the polynucleotide comprises an extended methylation-free CpG island containing bidivergent promoters adjacent to at least one further sequence comprising a methylation-free CpG island.

Preferably the polynucleotide comprises the human RNP CpG island/promoter region and a DNA fragment in the range 100bp to 3.0kb spanning the human β-actin CpG island/promoter region.

Alternatively, as described the polynucleotide may comprise the sequence of Figure 7 or a fragment thereof in either orientation. Preferably the polynucleotide comprises one or more promoter sequences.

It is known in the art that initiation of transcription may, under some circumstances, be inhibited by read-through transcripts from upstream promoters (Youssoufian and Lodish (1993) Transcriptional inhibition of the murine erythropoietin receptor gene by an upstream repetitive element, Mol Cell Biol 13 (1) 98-104). Therefore, as described, one or more of the promoter sequences are mutated in such a way that they are incapable of initiating transcription.

Preferably the promoter is selected from CMV, EF-la, RSV LTR, or HIV2 LTR or combinations of sequences derived therefrom. More preferably the promoter is a CMV promoter. Most preferably it is the mouse CMV promoter.

Preferably the polynucleotide comprises at least one sequence which is artificially synthesised.

Also described is a vector comprising the polynucleotide.

Preferably said vector is an expression vector adapted for eukaryotic gene expression.

Typically said adaptation includes, by example and not by way of limitation, the provision of transcription control sequences (promoter sequences) which mediate cell/tissue specific expression.

Promoter and enhancer are terms well-known in the art and include the following features which are provided by example only, and not by way of limitation. Promoters are 5', cis-acting regulatory sequences directly linked to the initiation of transcription. Promoter elements include so-called TATA box and RNA polymerase initiation selection (RIS) sequences which function to select a site of transcription initiation. These sequences also bind polypeptides which function, *inter alia,* to facilitate transcription initiation selection by RNA polymerase.

Enhancer elements are *cis* acting nucleic acid sequences often found 5' to the transcription initiation site of a gene (enhancers can also be found 3' to a gene sequence or even located in intronic sequences and is therefore position independent). Enhancers function to increase the rate of transcription of the gene to which the enhancer is linked. Enhancer activity is responsive to *trans* acting transcription factors (polypeptides) which have been shown to bind specifically to enhancer elements. The binding/activity of transcription factors is responsive to a number of environmental cues which include, by way of example and not by way of limitation, intermediary metabolites (eg glucose), environmental effectors (eg heat,). (See Eukaryotic Transcription Factors, by David S. Latchman, Academic Press Ltd, San Diego)

Adaptations also include the provision of selectable markers and autonomous replication sequences which both facilitate the maintenance of said vector in either the eukaryotic cell or prokaryotic host. Vectors which are maintained autonomously are referred to as episomal vectors. Episomal vectors are desirable since they are self-replicating and so persist without the need for integration. Episomal vectors of this type are described in WO98/07876.

Adaptations which facilitate the, expression of vector encoded genes include the provision of transcription termination/polyadenylation sequences. This also includes the provision of internal ribosome entry sites (IRES) which function to maximise expression of vector encoded genes arranged in bicistronic or multi-cistronic expression cassettes.

These adaptations are well-known in the art. There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol III IRL Press, Oxford UK; DNA Cloning: F M Ausubel et al, Current Protocols in Molecular Biology, John Wiley & Sons, Inc. (1994).

Preferably the vector comprises an expressible gene operably-linked to a promoter and the polynucleotide.

Preferably the vector is an episomal or integrating vector.

Alternatively, the vector of the present invention is a plasmid.

Alternatively, the vector may be a virus, such as an adenovirus, adeno-associated virus, a herpesvirus, vaccinia virus, lentivirus or other retrovirus.

Preferably the operably-linked gene is a therapeutic nucleic acid sequence.

Preferably the vector comprises two sites for insertion of open reading frames to be expressed, each transcribed from a distinct promoter, said promoters being arranged so as to transcribe divergently and both promoters being operably-linked to an artifically-constructed UCOE situated between them, and wherein said UCOE has been so constructed as to be effective in both orientations. This is particularly useful for the production of proteins that comprise two or more polypeptide chains, including, but not limited to, immunoglobulins. The insertion sites in the vector may be inserted with nucleic acid encoding different polypeptides of interest, including, but not limited to an open reading frame encoding an immunoglobulin heavy and an immunoglobulin light chain.

Preferably the vector comprises the sequence of Figure 2, the CMV promoter, a multiple cloning site, a polyadenylation sequence and genes encoding selectable markers under suitable control elements. It will be apparent to one of skill in the art that the UCOE can be inserted in both orientations.

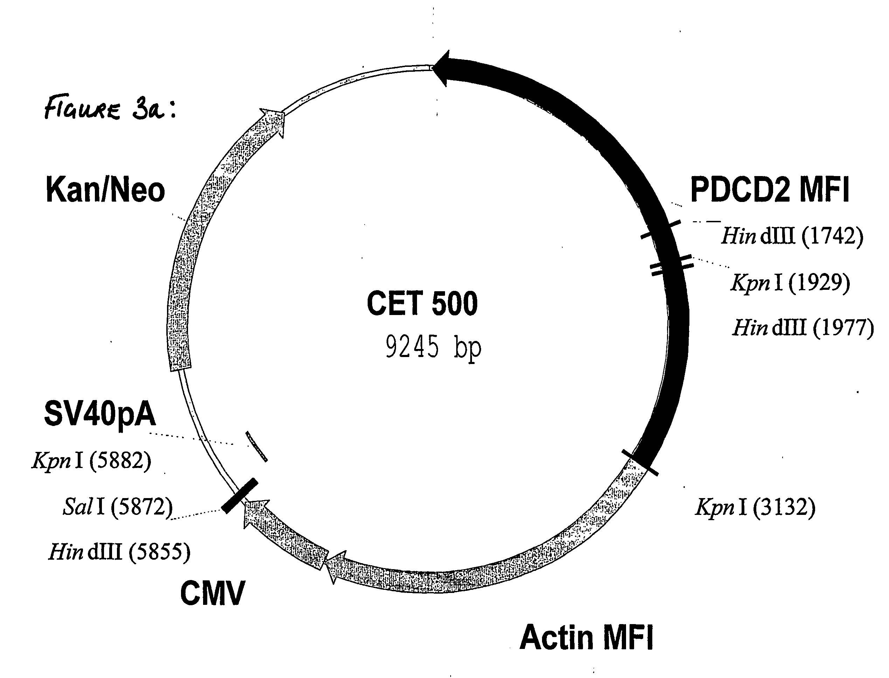

Preferably the vector comprising the artificial UCOE is CET 500 as shown schematically in Figure 3a.

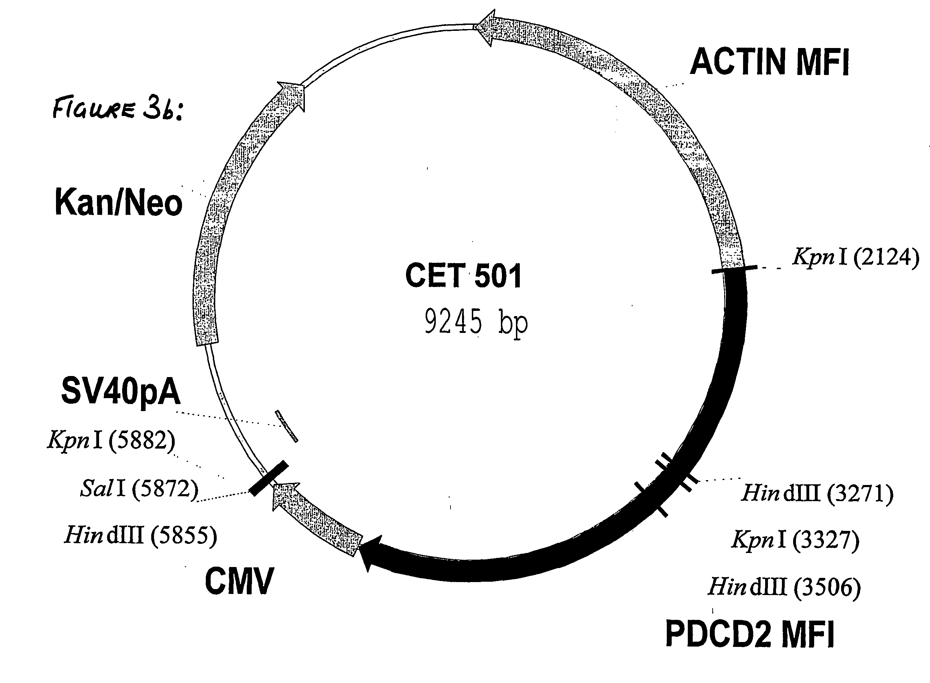

As described, the vector may be CET 501 as shown in Figure 3b.

Alternatively, the vector of any of claims comprises the sequence of Figure 7, the CMV promoter, a multiple cloning site, a polyadenylation sequence and genes encoding selectable markers under suitable control elements. Similarly, it will be apparent to one of skill in the art that the artificial UCOE can be inserted in both orientations.

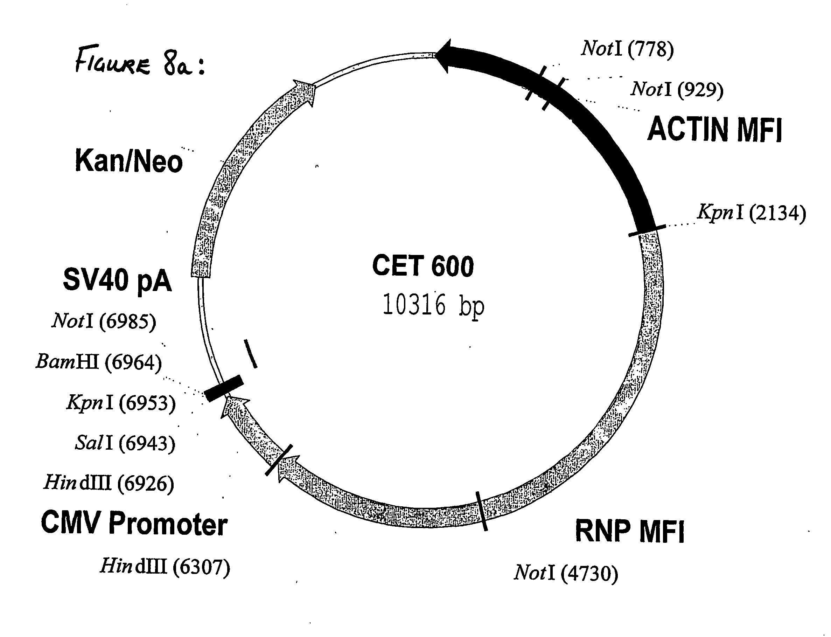

As described, the vector comprising the artificial UCOE may be CET 600 as shown schematically in Figure 8a.

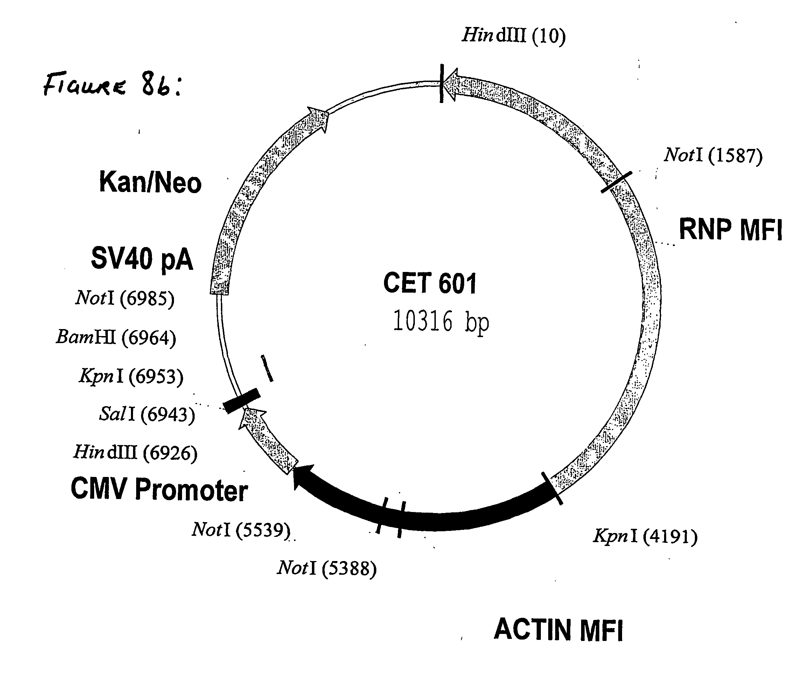

As described, the vector is CET 601 as shown schematically in Figure 8b.

Also described is a host cell that is transfected with the vector of the present invention.

Also described is the polynucleotide, vector or the host cell for use in therapy.

Also described is use of the polynucleotide, vector or host cell in the manufacture of a composition for use in gene therapy.

Also described is a method of treatment, comprising administering to a patient in need of such treatment a pharmaceutical effective amount of the polynucleotide, vector or host cell of the present invention. Preferably the patient is suffering from a disease treatable by gene therapy.

Also described is a pharmaceutical composition comprising the polynucleotide and/or the vector and/or host cell, optionally in admixture with a pharmaceutically acceptable carrier or diluent, for therapy to treat a disease or provide the cells of a particular tissue with an advantageous protein or function.

As described, the polynucleotide vector or host cell or the pharmaceutical composition may be administered via a route which includes systemic intramuscular, intravenous, aerosol, oral (solid or liquid form), topical, ocular, rectal, intraperitoneal and/or intrathecal and local direct injection.

The exact dosage regime will, of course, need to be determined by individual clinicians for individual patients and this, in turn, will be controlled by the exact nature of the protein expressed by the gene of interest and the type of tissue that is being targeted for treatment.

The dosage also will depend upon the disease indication and the route of administration. The number of doses will depend upon the disease, and the efficacy data from clinical trials.

The amount of polynucleotide or vector DNA delivered for effective gene therapy according to the invention will preferably be in the range of between 50 ng-1000 $\mu$g of vector DNA/kg body weight; and more preferably in the range of between about 1100 $\mu$g vector DNA/kg.

As described the polynucleotide, vector or host cell may be administered to a mammal for *in vivo* cell uptake, or an *ex vivo* approach may be utilised whereby cells are removed from an animal, transduced with the polynucleotide or vector, and then re-implanted into the animal. The liver, for example, can be accessed by an *ex vivo* approach by removing hepatocytes from an animal, transducing the hepatocytes *in vitro* and re-implanting the transduced hepatocytes into the animal (e. g., as described for rabbits by Chowdhury et al., Science 254:1802-1805,1991, or in humans by Wilson, Hum. Gene Ther. 3: 179-222, 1992). Such methods also may be effective for delivery to various populations of cells in the circulatory or lymphatic systems, such as erythrocytes, T cells, B cells and haematopoietic stem cells.

Also described is a mammalian model for determining the efficacy of gene therapy using the described polynucleotide, vector or host cell of the invention. The mammalian model comprises a transgenic animal whose cells contain the vector of the present invention. Methods of making transgenic mice (Gordon et al., Proc. Natl. Acad Sci. USA 77: 7380 (1980); Harbers et al., Nature 293:540 (1981) ; Wagner et al., Proc. Natl. Acad. Sci. USA 78: 5016 (1981) ; and Wagner et al, Proc. Natl. Acad. Sci. USA 78: 6376 (1981), sheep pigs, chickens (see Hammer et al., Nature 315: 680 (1985)), etc., are well-known in the art and are contemplated for use. Such animals permit testing prior to clinical trials in humans.

Transgenic animals containing the polynucleotide also may be used for long-term production of a protein of interest.

Also described is the use of the polynucleotide and/or vector and/or host cell in a cell culture system in order to obtain a desired gene product.

Suitable cell culture systems are well known in the art and are fully described in the body of literature known to those skilled in the art.

Also described is the use of the polynucleotide to increase the expression of an endogenous gene comprising inserting the polynucleotide into the genome of a cell in a position operably associated with the endogenous gene thereby increasing the level of expression of the gene.

Also described is the use of the polynucleotide in producing transgenic plants.

The generation of transgenic plants which have increased yield, resistance, etc. are well known to those skilled in the art. Also described are transgenic plant containing cells which contain the described polynucleotide. Some or all of the cells comprising the artificial UCOE may originate from plants.

Also described is a transgenic non-human animal containing cells which contain the polynucleotide.

Also described is the use of the described polynucleotide in functional genomics applications. Functional genomics relates principally to the sequencing of genes specifically expressed in particular cell types or disease states and now provides thousands of novel gene sequences of potential interest for drug discovery or gene therapy purposes. The major problem in using this information for the development of novel therapies lies in how to determine the functions of these genes. UCOEs can be used in a number of functional genomic applications in order to determine the function of gene sequences, for example:

(1) Using the described polynucleotide to achieve sustained expression of anti-sense versions of the gene sequences or ribozyme knockdown libraries, thereby determining the effects of inactivating the gene on cell phenotype.

(2) Using the described polynucleotide to prepare expression libraries for the gene sequences, such that delivery into cells will result in reliable, reproducible, sustained expression of the gene sequences. The resulting cells, expressing the gene sequences can be used in a variety of approaches to function determination and drug discovery. For example, raising neutralising antibodies to the gene product; rapid purification of the protein product of the gene itself for use in structural, functional or drug screening studies ; or in cell-based drug screening.

(3) Using the described polynucleotide in approaches involving mouse embryonic stem (ES) cells and transgenic mice. One of the most powerful functional genomics approaches involves random insertion into genes in mouse ES cells of constructs which only allow drug selection following insertion into expressed genes, and which can readily be rescued for sequencing (G. Hicks et al., Nature Genetics, 16,338-334). Transgenic mice with knockout mutations in genes with novel sequences can then readily be made to probe their function. At present this technology works well for the 10% of mouse genes which are well expressed in mouse ES cells.

Incorporation of UCOEs into the integrating constructs will enable this technique to be extended to identify all genes expressed in mice.

Also described is a method for the production of a polypeptide:

i) providing a cell transformed/transfected with a nucleic acid molecule;

ii) growing said cell in conditions conducive to the manufacture of said polypeptide; and

iii) purifying said polypeptide from said cell, or its growth environment.

The nucleic acid molecule may be a vector.

Preferably said vector encodes, and thus said polypeptide is provided with, a secretion signal to facilitate purification of said polypeptide.

Alternatively, further refinements to facilitate purification of expressed recombinant protein, such as affinity tags or epitopes, or enzymatic cleavage sites may be included.

## DETAILED DESCRIPTION OF THE INVENTION

The invention will now be described by way of example only and with reference to the accompanying figures wherein;

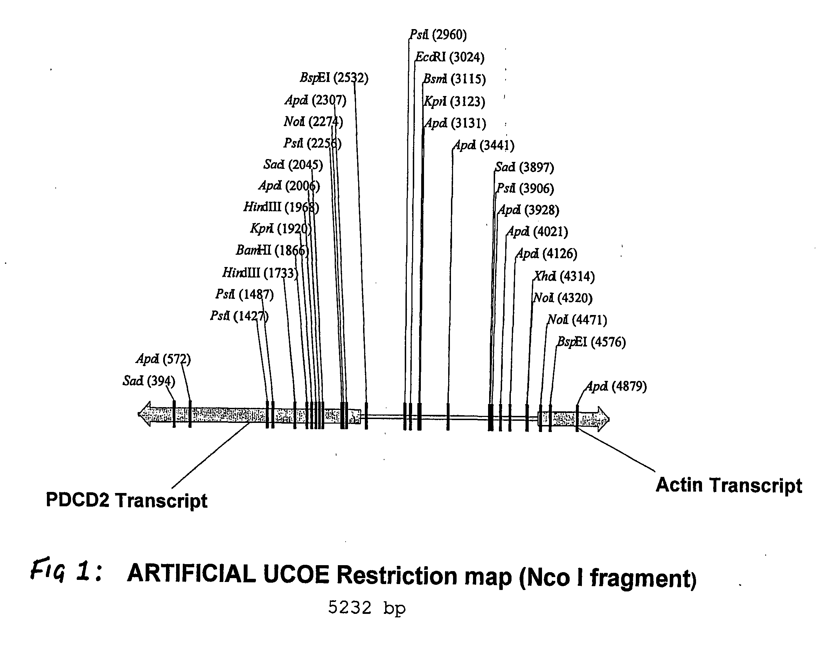

Figure 1 is a linear restriction map of the artificial UCOE *Nco* I fragment. The PDCD2 and actin transcripts are shown as shaded arrows, with the direction of the arrow depicting the direction of transcription from their respective promoters.

Figure 2 shows the nucleotide sequence of the PDCD2/Actin artificial UCOE *Nco* I fragment according to the invention.

Figures 3a and 3b depict plasmid maps of the PDCD2/Actin artificial UCOE-containing expression vectors.

Figure 3a shows CET 500, comprising the PDCD2/Actin artificial UCOE upstream of the CMV promoter and a multiple cloning site suitable for insertion of the open reading frame to be expressed. In this particular embodiment the plasmid backbone is from pEGFPN-1 and carries a kanamycin/ neomycin resistance gene.

Figure 3b shows CET 501, comprising the PDCD2/actin artificial UCOE in the reverse orientation.

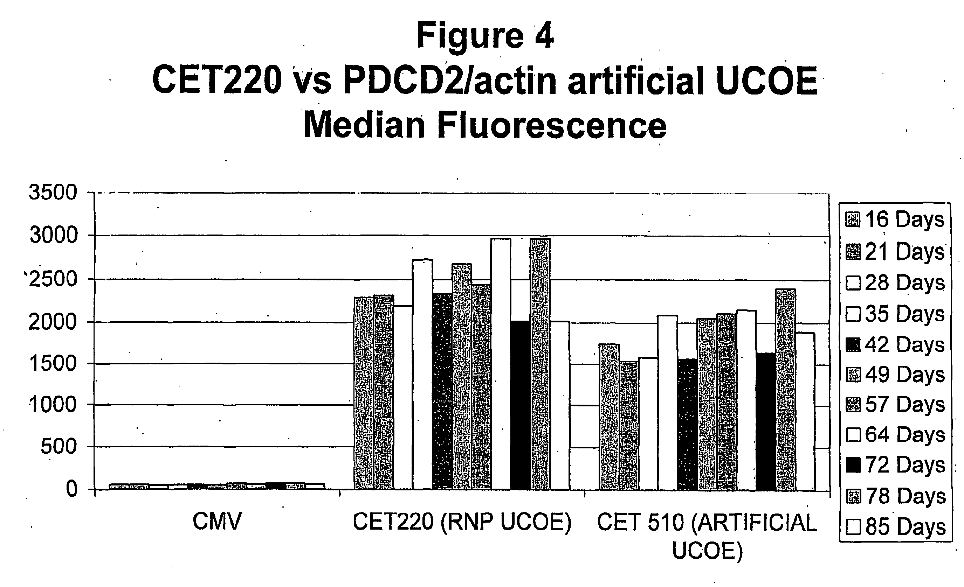

Figure 4 shows levels of expression of a reporter transgene (EGFP) in pools of stably-transfected CHO cells maintained under G418 selection over a period of 85 days. Expression was driven by the CMV promoter alone (CMV), or in combination with the 8kb RNP/HP-1 UCOE (CET 220) or artificial PDCD2/actin UCOE CET 510) and was measured as median fluorescence by FACS .

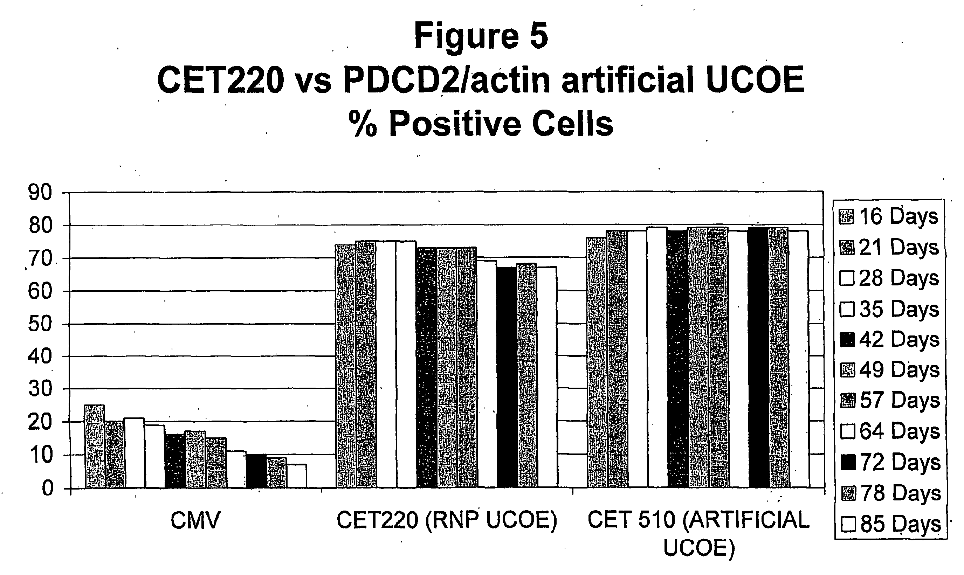

Figure 5 shows the proportion of cells expressing the transgene in the above experiment expressed as % positive cells on FACS analysis over a period of 85 days.

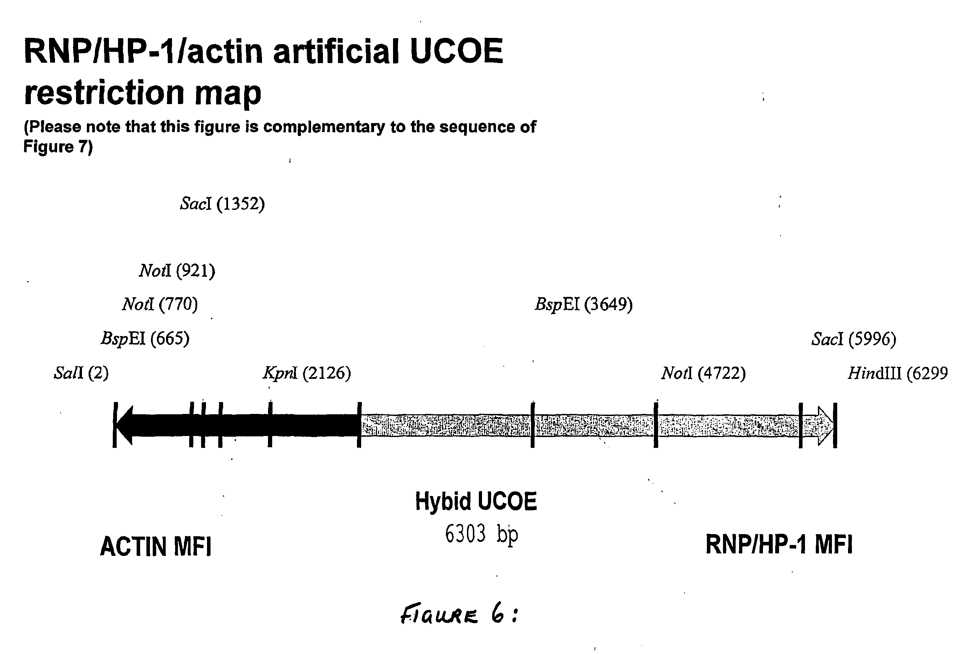

**Figure 6** is a linear restriction map of the RNP/HP-1/actin artificial UCOE fragment. The methylation-free islands are shown as shaded arrows, with the direction of the arrow depicting the direction of transcription from their respective promoters.

**Figure 7** shows the nucleotide sequence of the RNP/HP-1/actin artificial UCOE fragment according to the invention.

**Figure 8** depicts plasmid maps of RNP/HP-1/actin artificial UCOE-containing expression vectors.

**Figure 8a** shows CET 600, comprising the RNP/HP-1/actin artificial UCOE upstream of the CMV promoter and a multiple cloning site suitable for insertion of the open reading frame to be expressed. In this particular embodiment the plasmid backbone is from pEGFPN-1 and carries a kanamycin/ neomycin resistance gene.

**Figure 8b** shows CET 601, comprising the RNP/HP-1/actin artificial UCOE in the reverse orientation.

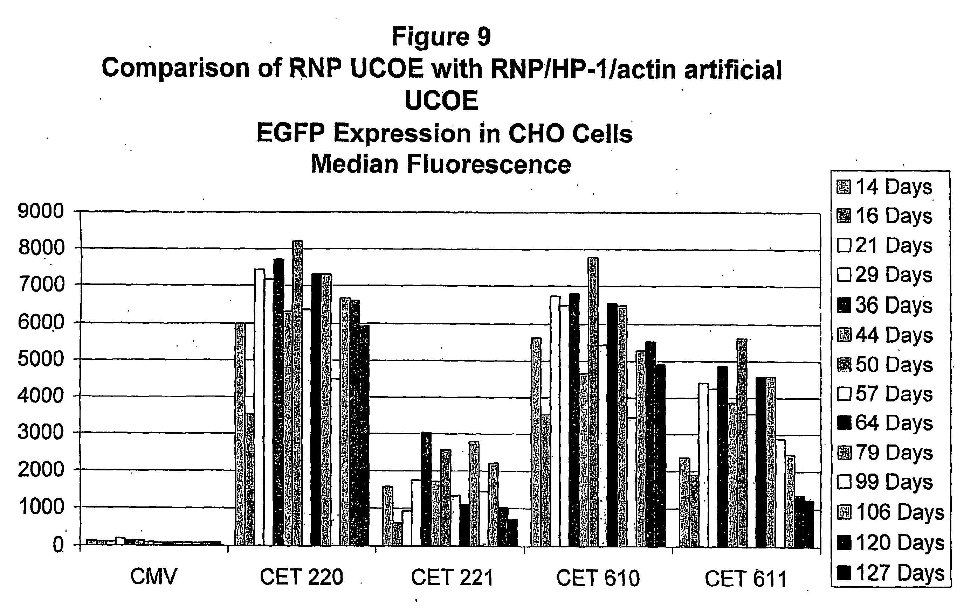

**Figure 9** shows levels of expression of a reporter transgene (EGFP) in pools of stably-transfected CHO cells maintained under G418 selection over a period of 127 days. Expression was driven by the CMV promoter alone (CMV), or in combination with the 8kb RNP UCOE in either forward (CET 220) or reverse (CET 221) or by RNP/HP-1/actin artificial UCOE in either forward (CET 610) or reverse (CET 611) orientation and was measured as median fluorescence by FACS. It will be understood that CET 610 is therefore the equivalent of CET 600, but containing EGFP as the inserted gene, and that CET 611 is the corresponding EGFP-containing equivalent of CET 601.

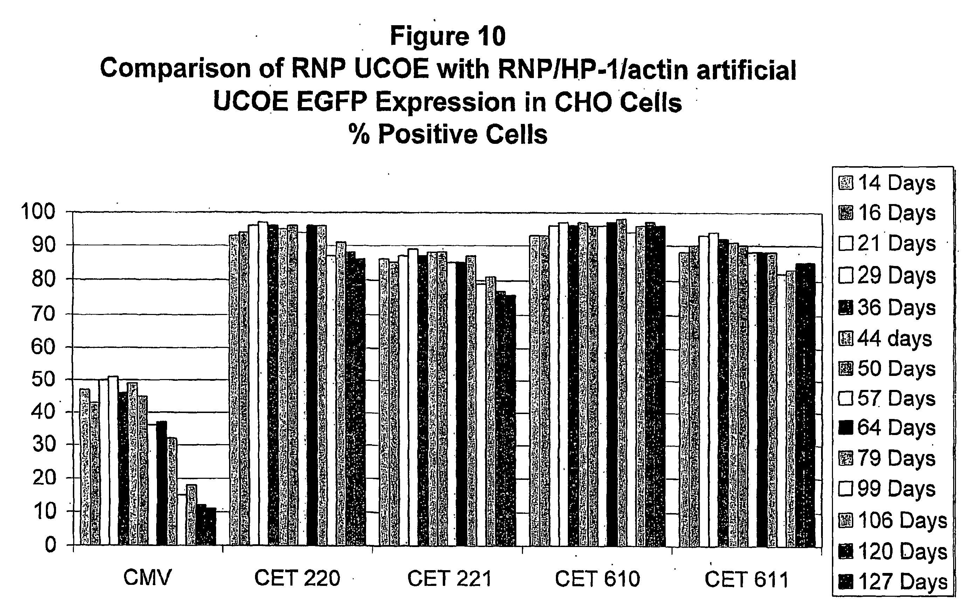

**Figure 10** shows the proportion of cells expressing the transgene in the above experiment expressed as % positive cells on FACS analysis over a period of 127 days.

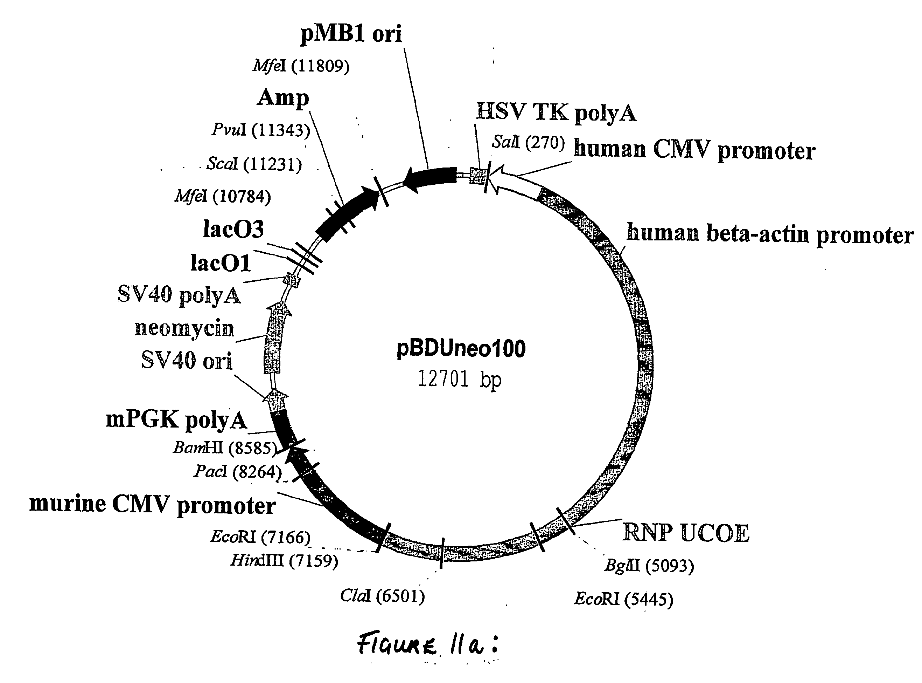

**Figures 11a-11d** depict plasmid maps of the bi-directional UCOE vectors for the expression of immunoglobulins.

## Example 1

### Heterologous combinations of UCOE fragments

We have found that the substitution of the *A2*/*HP1* fragments in the 4.0CMV and 8.0CMV constructs with the comparable region from the *TBP*/*B1* locus gave substantial increases in the level and stability of EGFP expression from the hCMV promoter. These findings in tissue culture cells provide evidence that regions of DNA encompassing an extended CpG island and divergently transcribed promoters are responsible for conferring an increase in the proportion of viable integration events, significantly improved transgene expression and a resistance to transcriptional silencing even from within centromeric heterochromatin.

Genomic regions encompassing CpG islands from housekeeping genes associated with a single promoter were combined. The construct comprised the 2kb CpG island from the 5' end of the human *β-actin* gene[37] joined to a 3.2kb fragment containing the CpG island from the 5' end of the human *PDCD2* gene. The promoters were divergently transcribed and their transcriptional start sites separated by 1.9kb.

The entire 5.2kb combination was then linked to an EGFP reporter gene driven by the CMV promoter (PDCD2/ACTIN). As a comparison, the *β-actin* CpG island/promoter region alone was also inserted upstream of the CMV-EGFP expression vector (ACTIN).

### Materials and Methods

With particular reference to Figure 2, the CMV promoter was removed from pEGFP-N1 by digestion with *Ase*I and *Nhe*I followed by blunting with T4 DNA polymerase and religation to produce pΔ-EGFP-N1. A β-Actin promoter EGFP fusion was constructed by digesting MA39 (HSACCYBB) with *Bsm*I and *Nco*I, isolating the appropriate fragment and blunting it with T4 DNA polymerase. This fragment was ligated into pΔ-EGFP-N1 that had been digested with *Age*I*,* blunted with T4 DNA polymerase and then digested with SmaI. This produced an in frame fusion of the first codon of the β-Actin gene with EGFP, expression of which was driven off the β-Actin promoter/MFI (methylation free island). The construct, pActin-EGFP, was shown to express EGFP in stably transfected CHOK1 cells.

To construct a vector with bi-directional promoters and an extended methylation free island, the PDCD2 gene and some of its promoter was initially removed from pCP2-TNN (approximately 160kb of the TBP locus in pCYPAC-2) by digestion with *Swa*I and *Bsp*EI and sub cloned into pBluescriptKS+ that had been digested with *Eco*RV and *Xma*I (pPDCD2-KS). As this vector did not contain the whole of the PDCD2 methylation free island, the remaining 5' region of the PDCD2 methylation free island, which also contained the promoter, was obtained by PCR from pCP2-TNN using the following priers 5'GCGGTACCAAGGGCATTCTGAAGTTAACC-3', 5-AGCTCCACAGGCCTGG-3'. The PCR product was then digested with *Kpn*I (site generated with PCR primers) and *Stu*I (internal site), pActin-EGFP was digested with *Sal*I and *Kpn*I, and the PDCD2 gene was removed from pPDCD2-KS by digestion with *Sal*I and *Stu*I. All three fragments were ligated together to create pPDCD2-Actin-EGFP.

The region containing the methylation free island of pF-PDCD2-Actin-EGFP was removed as an *Nco* I fragment (approximately 5.2kb), this was blunted with T4 DNA polymerase and then ligated, in both orientations, into pEGFPN-1 that had been digested with *Ase* I and then blunted. These vectors were called CET 510 (UCOE in forward orientation) and CET511 (UCOE in reverse orientation). The corresponding empty expression vectors with no transgene inserted into the multiple cloning sites were termed CET 500 and CET 501, respectively (see Figure 3).

Constructs were linearized with *Pvu* I, transfected into CHO-K1 cells and selected under G418 selection (0.6mg/ml) for both experiments.

It will be understood that one of skill in the art may adapt these procedures for preparation and testing of other polynucleotides of the invention.

## Results

As shown in Figures 4 and 5, the artificial UCOE construct gave levels of reporter gene expression that were comparable with those achieved with the 8kb RNP/HP-1 UCOE in terms of median fluorescence, and slightly better in terms of the proportion of cells expressing over the time course of the experiment.

### Example 2

### Heterologous combinations of UCOE and methylation-free CpG island fragments

### Materials and Methods

The actin methylation-free island was removed from pActin-EGFP by digestion with *Nco* I, blunted followed by digestion with *Kpn* I. The RNP 4kb fragment was removed from CET 20 by digestion with *Kpn* I and *Hind* III. These two fragments were then ligated into pBKS which had been digested with *Cla* I, blunted and then cut with *Hind* III. This gave the artificial UCOE in pBKS.

The artificial UCOE was then removed from pBKS by digestion with *Sal* I and *Hind* III, blunted and ligated into pEGFP-N1 that had been digested with *Ase* I and blunted. The UCOE was inserted in both orientations to create CET 610 and CET 611 respectively. The corresponding expression vectors with no transgene inserted into multiple cloning sites were termed CET 600 and CET 601, respectively (see Figure 8).

Constructs were linearised and transfected into CHO-K1 cells and selected under G418 selection (0.6mg/ml) for the duration of the experiments.

### Results

The effects of the artificial UCOE (in both orientations) on levels of transgene expression were assessed by comparison with results obtained with CMV promoter alone, and the 8kb RNP/HP-1 UCOE (in both orientations). Figure 9 shows that, in terms of median fluorescence on FACS analysis, the CMV promoter alone gave very low levels of expression. Both the RNP UCOE (CET 220) and artificial UCOE (CET 600) gave greatly increased (30-fold) expression in the forward orientation and were comparable. However, a directional bias seen with the RNP UCOE was less marked with the artificial UCOE, i.e. in the reverse orientation the artificial UCOE was superior, although still slightly less good than either UCOE in the forward orientation. This was true for both overall level of expression and ability to maintain expression over a prolonged period (more than 120 days).

When the same experiment was analysed interms of proportion of cells maintaining expression over time, both UCOEs, in both orientations, gave consistently better results than CMV alone (approximately twice as many cells expressing in the early part of the experiment). This difference became more marked over longer periods, with the CMV only population progressively losing expressing cells, until by 120 days only approximately 10% cells were still expressing. This contrasts with maintenance at approximately 90% for both UCOEs in the forward orientation, and only slightly lower levels (75-85%) for the reverse orientations by the end of the experiment.

### Example 3

**Co-expression of two genes on the same vector**

Efficient functional antibody production requires appropriately balanced expression of the heavy and light chains. Transfection of the two chains on separate plasmids makes the maintenance of an equal copy number difficult and provides the potential for transcriptional interference between the genes if the vectors integrate close to one another in the genome.

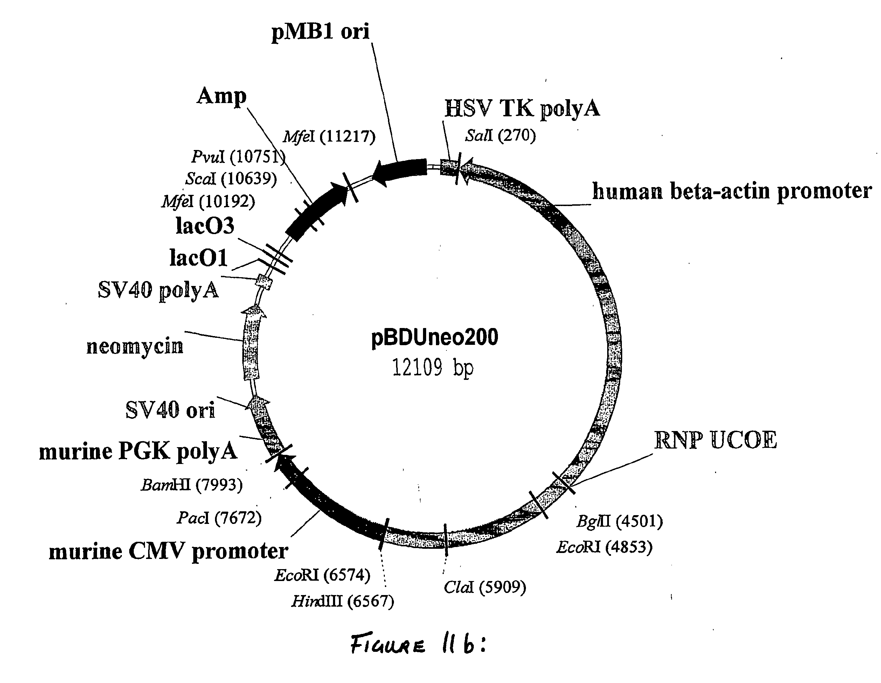

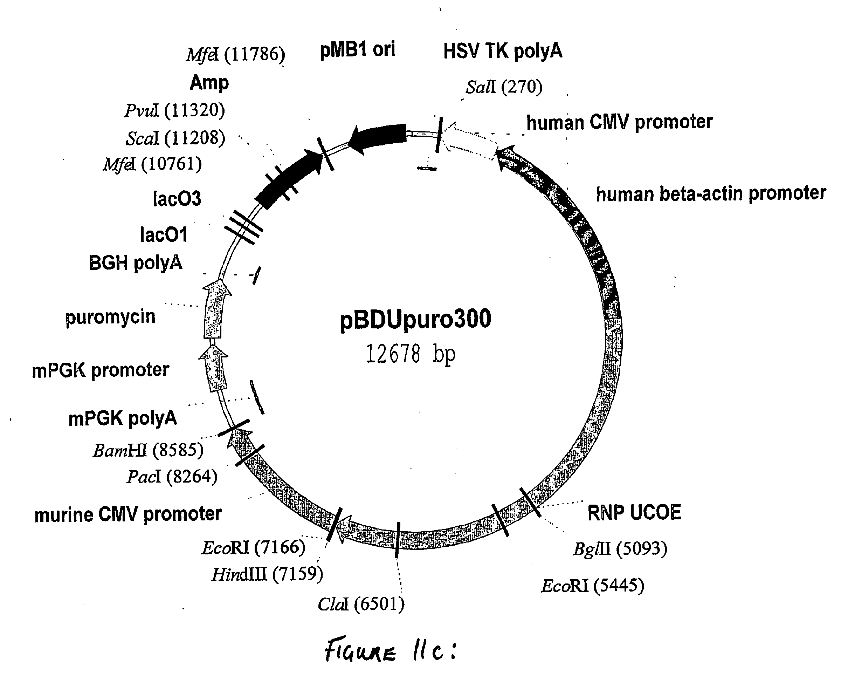

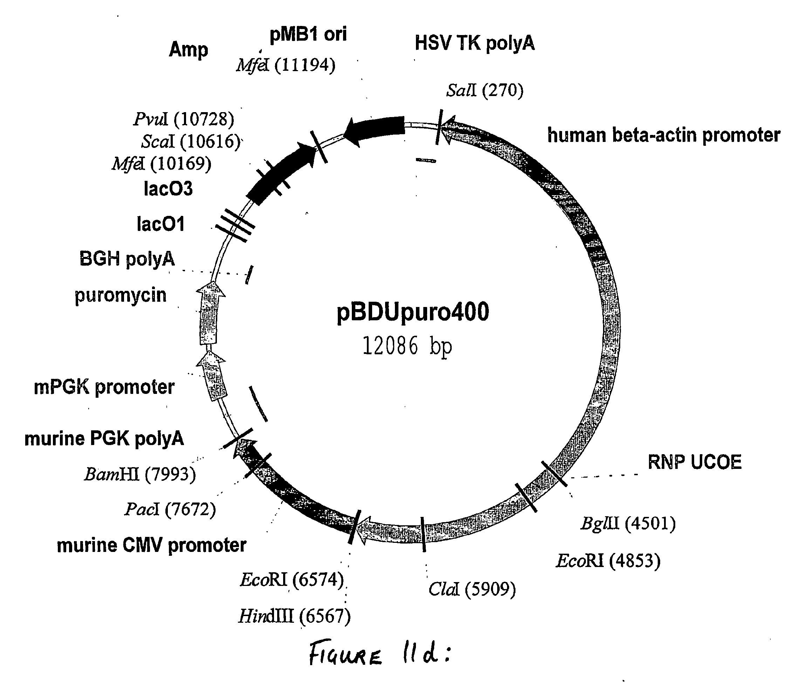

A series of new vectors for the co-expression of two genes on the same vector have been constructed to compare neo versus puro as resistance markers and hCMV, beta actin or mCMV promoters to drive light or heavy chain expression (figure 11).

**Material and Methods**

The two *Sfi* I sites of pORT1 (Cobra) were changed to *Mfe* I sites by introduction of adapter molecules comprised of annealed oligos Mfe.F, 5'-AACAATTGGCGGC and Mfe.R, 5'-GCCAATTGTTGCC.

The HSV TK polyA site was amplified from pVgRXR (Invitrogen) with primers TK.F, 5'ACGCGTCGACGGAAGGAGACAATACCGGAAG and TK.R, 5'CCGCTCGAGTTGGGGTGGGGAAAAGGAA. The *Sal* I to *Xho* I fragment was inserted into the *Sal* I site. The murine PGK polyA site was amplified from male BALB/c genomic DNA (Clontech) using primers mPGK.F, 5'-CGGGATCCGCCTGAGAAAGGAAGTGAGCTG and mPGK.R, 5'GAAGATCTGGAGGAATGAGCTGGCCCTTA, and the *Bam*H I to *Bgl* II fragment was cloned into the *Bam*H I site.

The *Ase* I to *Sal* I fragment of pcDNA3.1 containing the *neo* expression cassette was treated with T4 DNA polymerase, ligated to *Spe* I linkers (5'-GACTAGTC). The *Spe* I fragment was then cloned into the *Spe* I site to give pORTneoF or the *EcoR* I to *Not* I fragment of CET700 (Cobra) carrying the puromycin resistance cassette was treated with T4 DNA polymerase, ligated to *Xba* I linkers, and the *Xba* I fragment was cloned into the *Xba* I site to give pORTpuroF.

The *Hind* III to *Bam*H I murine CMV promoter fragment from pCMVEGFPN-1 (Cobra) was subcloned into the *Hind* III to *Bam*H I sites of the Hybrid UCOE in BKS+ (Cobra). The human CMV promoter was then amplified from plasmid pIRESneo (Clontech) using primers hCMVF, 5'-CTCGAGTTATTAATAGTAATCAATTACGGGGTCAT and hCMVR, 5'-GTCGACGATCTGACGGTTCACTAAACCAGCTCT and the *Xho* I to *Sal* I fragment was cloned into the *Sal* I site. The *Bam*H I to *Sal* I fragment was then cloned into the *Bam*H I to *Sal* I sites of pORTneoF to give pBDUneo100, or into pORTpuroF to give pBDUpuro300.

The two ATG codons upstream of the *Sal* I cloning site in the Hybrid UCOE in BKS+ were altered by site-directed mutagenesis and the *Bam*H I to *Sal* I fragment was cloned into the *Bam*H I to *Sal* I sites of pORTneoF to give pBDUneo200, or into pORTpuroF to give pBDUpuro400.

Human antibody light chain coding sequences were cloned into either the *Bam*H I or *Sal* I sites of all four bi-directional UCOE vectors (pBDUneo100, pBDUneo200, pBDUpuro300 and pBDUpuro400), followed by immunoglobulin heavy chain coding sequence at the remaining *Bam*H I or *Sal* I cloning site to give pBDUneo112, pBDUneo121, pBDUneo212, pBDUneo221, pBDUpuro112, pBDUpuro121, pBDUpuro212 and pBDUpuro221. All eight antibody expression constructs were transfected into CHO-K1 cells using Lipofectamine (Invitrogen) following the manufacturer's instructions, and selected with 500 μg/ml G418 (*neo* vectors) or 12.5 μg/ml puromycin (*puro* vectors).

Pools were selected and antibody production rates compared between the different constructs to determine the optimal promoter and selectable marker combination for antibody expression in CHO cells.

**Results**

Table 1 demonstrates that vectors containing the light chain expressed from the murine CMV promoter gave the best expression of the antibody. No significant difference was observed between production rates obtained with vectors containing the G418 or puromycin-resistance cassettes. The production rate from the pool of a co-transfection experiment performed separately is compared. Clones from this pool were isolated with production rates of 3-18pg/cell/day. However, clones above 5pg/cell/day were unstable and rapidly decreased in expression or stopped producing. Clones expressing approx. 5pg/cell/day were used for initial fermentation experiments. These preliminary indications are very encouraging that higher production rates will be observed in clones isolated from the bi-directional UCOE vector transfectants.

Table 1: Expression of hAb1 (IgG4) from Bi-directional UCOE vectors (CHO-K1 pools)

**CHO-K1 POOLS**

| Vector pg/cell/day) | H3 Promoter | K1 Promoter | Production Rate |
|---|---|---|---|
| pBDUneo112 | murine CMV | human CMV | 0.3 |
| pBDUneo121 | human CMV | murine CMV | 1.5 |
| pBDUneo212 | murine CMV | human beta-actin | 0.06 |
| pBDUneo221 | human beta-actin | murine CMV | 1.3 |
| pBDUpuro312 | murine CMV | human CMV | 0.5 |
| pBDUpuro321 | human CMV | murine CMV | 1.4 |
| pBDUpuro412 | murine CMV | human beta-actin | 0.05 |
| pBDUpuro421 | human beta-actin | murine CMV | 2.3 |
| Cotransfection** | human CMV | human CMV | 0.7 |

**Contransfection was carried out previously using the same antibody genes each driven from 4kb UCOE CMV vectors (hydro and neo selection).

REFERENCES

1. Dillon, N. & Grosveld, F. Chromatin domains as potential units of eukaryotic gene function. Curr. Opin. Genet. Dev. 4, 260-264 (1994).

2. Higgs, D.R. Do LCRs open chromatin domains? Cell 95, 299-302 (1998).

3. Palmiter, R.D. & Brinster, R.L. Germline transformation of mice. Ann. Ref Genet. 20, 465-499 (1986).

4. Allen, N.D. et al. Transgenes as probes for active chromosomal domains in mouse development. Nature 333, 852-855 (1988).

5. Bonnerot, C., Grimber, G., Briand, P. & Nicolas, J.F. Patterns of expression of position-dependent integrated transgenes in mouse embryo. Proc. Natl. Acad Sci. USA 87:6331-6335 (1990).

6. Kioussis, D. & Festenstein, R. Locus control regions: overcoming heterochromatin-induced gene inactivation in mammals. Curr. Opin. Genet. Dev. 7, 614-619 (1997).

7. Pikaart, , M.J., Recillas-Targa, F. & Felsenfeld, G. Loss of transcriptional activity of a transgene is accompanied by RNA methylation and histone deacetylation and is prevented by insulators. Genes Dev. 12, 2852-2862 (1998).

8. Fussenegger, M., Bailey, J.E., Hauser, H. & Mueller, P.P Genetic optimization of recombinant glycoprotein production by mammalian cells. Trends Biotech. 17, 35-42 (1999).

9. Fraser, P. & Grosveld, F. Locus control regions; chromatin activation and transcription. Curr. Opin. Cell Biol. 10, 361-365 (1998).

10. Li, Q., Harju, S. & Peterson, K.R. Locus Control Regions: coming of age at a decade plus. Trends Genet. 15: 403-408 (1999).

11. Bulger, M. & Groudine, M. Looping versus linking: toward a model for long-distance gene activation. Genes Dev. 13, 2465-2477 (1999).

12. Grosveld, F. Activation by locus control regions? Curr. Opin. Genet. Dev. 9 152-157 (1999).

13. Bender, M.A., Bulger, M., Close, J. & Groudine, M. ß-globin Gene Switching and Dnase I Sensitivity of the Endogenous ß-globin Locus in Mice Do Not Require the Locus Control Region. Mol. Cell 5, 387-393 (2000).

14. Ortiz, B.D., Cado, D., Chen, V., Diaz, P.W. & Winoto, A. Adjacent DNA elements dominantly restrict the ubiquitous activity of a novel chromatin-opening region to specific tissues. EMBO J. 16, 5037-5045 (1997).

15. Ortiz, B.D., Cado, D. & Winoto, A. A new element within the T-cell receptor alpha locus required for tissue-specific locus control region activity. Mol. Cell. Biol. 19, 1901-1909 (1999).

16. Antequera, F. & Bird, A. Number of CpG islands and genes in human and mouse. Proc. Natl. Acad Sci. USA 90, 1195-11999 (1993).

17. Cross, S.H. & Bird, A.P. CpG islands and genes. Curr. Opin, Genet. Dev. 5, 309-314 (1995).

18. Tazi, J. & Bird, A. Alternative chromatin structure at CpG islands. Cell 60, 909-920 (1990).

19. Imbert, G., Trottier, Y., Bechmann, J., & Mandel, J.L. The gene for the TATA binding protein (TBP) that contains a highly polymorphic protein coding CAG repeat maps to 6q27. Genomics 21: 667-668 (1994).

20. Purrello, M. et al. Physical mapping at 6q27 of the locus for the TATA-box binding protein, the DNA binding subunit of TFIID and a component of SL1 and TFIIIB, strongly suggests that it is single copy in the human genome. Genomics 22, 94-100 (1994).

21. Trachtulec, Z et al. Linkage of TATA-binding protein and proteasome subunit C5 genes in mice and humans reveals synteny conserved between mammals and invertebrates. Genomics 44: 1-7 (1997).

22. Owens, G.P., Hahan, W.E., & Cohen, J.J. Identification of mRNAs associated with programmed cell death in immature thymocytes. Mol. Cell. Biol. 11, 4177-4188 (1991).

23. Chalut, C., Gallois, Y., Poterszman, A., Moncollin, V. & Egly, J.-M. Genomic structure of the human TATA-box-binding protein (TBP). Gene 161,277-282 (1995).

24. Schmidt, E.E. & Schibler, U. High accumulation of components of the RNA polymerase II transcription machinery in rodent spermatids. Development 121, 2373-2383 (1995).

25. Biamonti, G., Ruggiu, M., Saccone, S., Della Valle, G. & Riva, S. Two homologous genes, originated by duplication, encode the human hnRNP proteins A2 and A1. Nucl. Acids Res. 22, 1996-2002 (1994).

26. Kozu, T., Henrich, B. & Schafer, K.P. Structure and expression of the gene (HNRPA2B1) encoding the human hnRNP protein A2/B1. Genomics 25, 365-371 (1995).

27. Ye, Q. & Worman, H.J. Interaction between an integral protein of the nuclear envelope inner membrane and human chromodomain proteins homologous to Drosophila HP1. J. Biol. Chem. 271, 14653-14656 (1996).

28. James, T.C. & Elgin, S.C.R. Identification of a nonhistone chromosomal protein associated with heterochromatin in Drosophila melanogaster and its gene. Mol. Cell. Biol. 6, 3861-3872 (1986).

29. Singh, P.B., et al. A sequence motif found in a drosophila heterochromatin protein is conserved in animals and plants. Nucl. Acids Res. 19, 789-794 (1991).

30. Gilliand, G., Perrin,m S., Blanchard, K. & Bunn, H.F. Analysis of cytokine Mrna and DNA: detection and quantitation by competitive polymerase chain reaction. Proc. Natl. Acad. Sci. USA 87, 2725-2729 (1990).

31. Furth, P.A. Hennighausen, L., Baker, C., Beatty, B. & Woychick, R. The viarability in activity of the universally expressed human cytomegalovirus immediate early gene 1 promoter/enhancer in transgenic mice. Nucl. Acids Res. 19 6205-6208 (1991).

32. Ray, P. et al. Ectopic expression of a c-kitW42 minigene in transgenic mice: recapitulation of W phenotypes and evidence for c-kit function in melanoblast progenitors. Genes Dev. 5, 2265-2273 (1991).

33. Yamashita, T. et al High level expression of human $\alpha$-fetoprotein in transgenic mice. Biochem, Biophys. Res.

Comm. 191, 715-720 (1993).

34. Milot, E. et al. Heterochromatin effects on the frequency and duration of LCR-mediated gene transcription. Cell 87, 104-114 (1996).

35. Festenstein, R. et al. Locus control region function and heterochromatin-induced position effect variegation. Science 271, 1123-1125 (1996).

36. Sabbattini, P., Georgiou, A., Sinclaire, C. & Dillon, N. Analysis of mice with single and multiple copies of transgenes reveals a novel arrangement for the λ5-V-preBI locus control region. Mol. Cell. Biol. 19, 671-679 (1999).

37. Ng, S.Y. et al. Evolution of the functional human ß-actin gene and its multi-pseudogene family: conservation of noncoding regions and chromosomal dispersion of pseudogenes. Mol. Cell. Biol. 5, 2720-2732 (1985).

38. Pravtcheva, D.D., Wise, T.L., Ensor, N.J. & Ruddle, F.H. Mosaic expression of an HPRT transgene integrated in a region of Y heterochromatin. J. Exp. Zool. 268, 452-468 (1994).

39. Bell, A.C. & Felsenfeld, G. Stopped at the border: boundaries and insulators. Curr. Opin. Genet. Dev. 9, 191-198 (1999).

40. Winston, J.H., Hong, L., Datta, S.K. & Kellems, R.E. An intron 1 regulatory region from the murine adenosine deaminase gene can activate heterologous promoters for ubiquitous expression in transgenic mice. Somat. Cell Mol. Genet. 22, 261-278 (1996).

41. Hart, C.M. & Laemmli, U.K. Facilitation of chromatin dynamics by SARs. Curr. Opin. Genet. Develop. 8, 519-525 (1998).

42. Klehr, D., Maass, K. & Bode, J. Scaffold-attached regions from the human interferon ß domain can be used to enhance the stable expression of genes under the control of various promoters. Biochemistry 30, 1264-1270 (1991).

43. McKnight, R.A., Shamay, L., Sankaran, L., Wall, R.J. & Henninghausen, L. Matrix-attachment regions can impart position-independent regulation of a tissue-specific gene in transgenic mice. Proc. Natl. Acad Sci. USA 89, 6943-6947 (1992).

44. Van Drunen, C.M., et al. A bipartite sequence element associated with matrix/scaffold attachment regions. Nucl. Acids Res. 27, 2924-293 0 (1999).

45. Verma, I.M. & Somia, N. Gene Therapy - promises, problems and prospects. Nature 389: 239-242 (1997).

46. Brown, S.A. & Kingston, R.E. Disruption of downstream chromatin directed by a transcriptional activator. Genes Dev. 11.3 116-3121 (1997).

47. Orphanides, G., LeRoy, G., Chang, C.H., Luse, D.S. & Reinberg, D. FACT, a factor that facilitates transcript elongation through nucleosomes. Cell 92, 105-116 (1998).

48. Schnitzler, G., Sif, S. & Kingston, R.E. Human SWI/SNF interconverts a nucleosome between its base state and a stable remodelled state. Cell 94, 17-27 (1998).

49. Travers, A. Chromatin modification by DNA tracking. Proc. Natl. Acad. Sci. USA 96, 13634-13637 (1999).

50. Ashe, H.L., Monks, J., Wijgerde, M., Fraser, P. & Proudfoot, N.J. Intergenic transcription and transinduction of the human ß-globin locus. Genes Dev 11, 2494-2509 (1997).

51. Rogan, D.F., Cousins, D.J. & Staynov, D.Z. Intergenic transcription occurs throughout the human IL-4/IL-13 gene cluster. Biochem, Biophys, Res. Commun. 255, 556-561 (1999).

52. Gribnau, J., Diderich, K., Pruzina, S., Calzorlari, R. & Fraser P. Intergenic Transcription and Developmental Remodeling of Chromatin Subdomains in the Human ß-globin Locus. Mol. Cell 5, 377-386 (2000).

53. Vyas, P. et al. Cis-acting sequences regulating expression of the human α-globin cluster lie within constitutively open chromatin. Cell 69, 781-793 (1992).

54. Ioannou, P.A. et al. A new bacteriophage P1-derrived vector for the propagation of large human DNA fragments. Nature Gene. 6, 84-89 (1994).

55. Raguz, S. et al. Muscle-specific locus control region activity associated with the human desmin gene. Dev. Biol. 201, 26-42 (1998).

56. Dillon, N. & Grosveld, F. Transcriptional analysis using transgenic animals, in Gene Transcription: A practical approach (eds Hames, B. D. & Higgins, S.J.) 153-188 (IRL Press, Oxford, 1993).

57. Morgenstern, J.P. & Land, H. Advanced mammalian gene transfer: high titre retroviral vectors with multiple drug selection markers and a complementary helper-free packaging cell line. Nucl. Acids Res 18, 3587-3595 (1990).

58. Horz, W. & Altenburger, W. Nucleotide sequence of mouse satellite DNA. Nucl. Acids Res. 9, 683-696 (1981).

59. Monfouilloux, S. et al. Recent human-specific spreading of a subtelomeric domain. Genomics 51, 165-176 (1998).

**Claims**

1. A method for aiding balanced expression of two divergently transcribed expression units when inserted for co-expression at two sites in the same vector, the method comprising:

   (i) providing two combined extended methylation-free CpG islands from different housekeeping genes in the form of a chimeric polynucleotide comprising the sequence of Figure 2 or Figure 7, wherein each extended methylation-free CpG island has a promoter;
   (ii) inserting the chimeric polynucleotide provided in step (i) between the vector sites selected for said expression units;

   whereby the promoters of said linked extended methylation-free CpG islands are orientated to drive divergent transcription of the expression units.

2. A method a claimed in claim 1 further comprising providing said expression units in the vector.

3. The method of claim 1 or claim 2, wherein the vector nucleotide sequence formed in step (ii) to direct transcription of said expression units includes a promoter selected from: cytomegalovirus (CMV), elongation factor-1 alpha (EF-1α), respiratory syncytial virus (RSV) LTR and human immunodeficiency 2 (HIV2) LTR or combinations thereof for initiating transcription.

4. The method of claim 3 wherein said nucleotide sequence includes a CMV promoter.

5. The method of claim 4, wherein the CMV promoter is a mouse CMV promoter.

6. A method according to any of the preceding claims wherein the divergently transcribed expression units express heavy and light chains of an immunoglobulin/antibody.

7. A chimeric polynucleotide comprising the sequence of Figure 2 or the sequence of Figure 7.

8. The polynucleotide of claim 7, wherein the nucleotide sequence further comprises a promoter selected from: cytomegalovirus (CMV), elongation factor-1 alpha (EF-1α), respiratory syncytial virus (RSV) LTR and human immunodeficiency 2 (HIV2) LTR or combinations thereof.

9. The polynucleotide of claim 8 wherein said nucleotide sequence further comprises a CMV promoter.

10. The polynucleotide of claim 9, wherein the CMV promoter is a mouse CMV promoter.

**11.** A vector comprising the polynucleotide of any of claims 7 to 10.

**12.** A vector as claimed in claim 11 obtainable by a method according to any one of claims 1 to 6.

**13.** A vector as claimed in claim 11 or claim 12 which is an expression vector wherein the promoters of said linked extended methylation-free CpG islands are orientated to drive divergent transcription of two expression units to be co-expressed.

**14.** A host cell containing a vector as claimed in any of claims 11 to 13.

**15.** A host cell as claimed in claim 14 which contains an expression vector according to claim 13.

**16.** A method for the production of a protein or polypeptide which comprises culturing a host cell according to claim 15 wherein said expression units are co-expressed and isolating said protein.

**17.** A method as claimed in claim 16 wherein the divergently transcribed expression units express heavy and light chains of an immunoglobulin and said immunoglobulin is isolated.

**18.** A polynucleotide or vector according to any of claims 7 to 13 for use as a medicament.

**Patentansprüche**

**1.** Verfahren zur Unterstützung einer ausgeglichenen Expression von zwei divergent transkribierten Expressionseinheiten bei Insertion an zwei Stellen in ein und demselben Vektor zur Koexpression, wobei das Verfahren Folgendes umfasst:

(i) Bereitstellen von zwei kombinierten erweiterten methylierungsfreien CpG-Inseln aus unterschiedlichen Haushaltsgenen in der Form eines chimären Polynukleotids, das die Sequenz von Figur 2 oder Figur 7 umfasst, wobei jede erweiterte methylierungsfreie CpG-Insel einen Promotor hat;
(ii) Insertieren des in Schritt (i) bereitgestellten chimären Polynukleotids zwischen den Vektorstellen, die für die Expressionseinheiten ausgewählt wurden;

wobei die Promotoren der verknüpften erweiterten methylierungsfreien CpG-Inseln zum Antreiben der divergenten Transkription der Expressionseinheiten ausgerichtet sind.

**2.** Verfahren nach Anspruch 1, das weiterhin das Bereitstellen der Expressionseinheiten in dem Vektor umfasst.

**3.** Verfahren nach Anspruch 1 oder 2, wobei die in Schritt (ii) gebildete Vektor-Nukleotidsequenz zum Steuern der Transkription der Expressionseinheiten einen Promotor, der aus den folgenden ausgewählt ist: Zytomegalie-Virus (ZMV), Elongationsfaktor-1-alpha (EF-1$\alpha$), LTR des respiratorischen Synzytial-Virus (RSV) und LTR des humanen Immundefizienz-Virus 2 (HIV2) oder Kombinationen davon, zum Initiieren der Transkription aufweist.

**4.** Verfahren nach Anspruch 3, wobei die Nukleotidsequenz einen ZMV-Promotor aufweist.

**5.** Verfahren nach Anspruch 4, wobei der ZMV-Promotor ein Maus-ZMV-Promotor ist.

**6.** Verfahren nach einem der vorhergehenden Ansprüche, wobei die divergent transkribierten Expressionseinheiten schwere und leichte Ketten eines Immunglobulins/Antikörpers exprimieren.

**7.** Chimäres Polynukleotid, das die Sequenz von Figur 2 oder die Sequenz von Figur 7 umfasst.

**8.** Polynukleotid nach Anspruch 7, wobei die Nukleotidsequenz weiterhin einen Promotor umfasst, der aus den folgenden ausgewählt ist: Zytomegalie-Virus (ZMV), Elongationsfaktor-1-alpha (EF-1$\alpha$), LTR des respiratorischen Synzytial-Virus (RSV) und LTR des humanen Immundefizienz-Virus 2 (HIV2) oder Kombinationen davon.

**9.** Polynukleotid nach Anspruch 8, wobei die Nukleotidsequenz weiterhin einen ZMV-Promotor umfasst.

**10.** Polynukleotid nach Anspruch 9, wobei der ZMV-Promotor ein Maus-ZMV-Promotor ist.

**11.** Vektor, der das Polynukleotid nach einem der Ansprüche 7 bis 10 umfasst.

**12.** Vektor nach Anspruch 11, der durch ein Verfahren nach einem der Ansprüche 1 bis 6 erhalten werden kann.

**13.** Vektor nach Anspruch 11 oder 12, der ein Expressionsvektor ist, wobei die Promotoren der verketteten erweiterten methylierungsfreien CpG-Inseln zum Antreiben der divergenten Transkription von zwei zu koexprimierenden Expressionseinheiten ausgerichtet ist.

**14.** Wirtszelle, die einen Vektor nach einem der Ansprüche 11 bis 13 enthält.

**15.** Wirtszelle nach Anspruch 14, die einen Expressionsvektor nach Anspruch 13 enthält.

**16.** Verfahren zur Produktion eines Proteins oder Polypeptids, wobei das Verfahren das Kultivieren einer Wirtszelle nach Anspruch 15, wobei die Expressionseinheiten koexprimiert werden, und das Isolieren des Proteins umfasst.

**17.** Verfahren nach Anspruch 16, wobei die divergent transkribierten Expressionseinheiten schwere und leichte Ketten eines Immunglobulins exprimieren und das Immunglobulin isoliert wird.

**18.** Polynukleotid oder Vektor nach einem der Ansprüche 7 bis 13 zur Verwendung als ein Arzneimittel.


**Revendications**

**1.** Procédé d'aide à l'expression équilibrée de deux modules d'expression transcrits de manière divergente lors de l'insertion pour la co-expression au niveau de deux sites dans le même vecteur, le procédé comprenant :

(i) fournir deux îlots CpG étendus combinés exempts de méthylation provenant de différents gènes domestiques sous la forme d'un polynucléotide chimère comprenant la séquence de la Figure 2 ou Figure 7, dans lequel chaque îlot CpG étendu exempt de méthylation a un promoteur ;
(ii) insérer le polynucléotide chimère fourni à l'étape (i) entre les sites vectoriels sélectionnés pour lesdits modules d'expression ;

moyennant quoi les promoteurs desdits îlots CpG étendus liés exempts de méthylation sont orientés pour entraîner la transcription divergente des modules d'expression.

**2.** Procédé selon la revendication 1, comprenant en outre la fourniture desdits modules d'expression dans le vecteur.

**3.** Procédé selon la revendication 1 ou la revendication 2, dans lequel la séquence nucléotidique de vecteur formée à l'étape (ii) pour diriger la transcription desdits modules d'expression inclut un promoteur sélectionné parmi : le cytomégalovirus (CMV), le facteur d'élongation-1 alpha (EF-1$\alpha$), la répétition terminale longue du virus syncytial respiratoire (RSV) et la répétition terminale longue du virus d'immunodéficience humaine 2 (VIH2) ou des combinaisons de ceux-ci pour initier la transcription.

**4.** Procédé selon la revendication 3, dans lequel ladite séquence nucléotidique inclut un promoteur de CMV.

**5.** Procédé selon la revendication 4, dans lequel le promoteur de CMV est un promoteur de CMV de souris.

**6.** Procédé selon l'une quelconque des revendications précédentes, dans lequel les modules d'expression transcrits de manière divergente expriment des chaînes lourdes et légères d'une immunoglobuline/d'un anticorps.

**7.** Polynucléotide chimère comprenant la séquence de la Figure 2 ou la séquence de la Figure 7.

**8.** Polynucléotide selon la revendication 7, dans lequel la séquence nucléotidique comprend en outre un promoteur sélectionné parmi : le cytomégalovirus (CMV), le facteur d'élongation-1 alpha (EF-1$\alpha$), la répétition terminale longue du virus syncytial respiratoire (RSV) et la répétition terminale longue du virus d'immunodéficience humaine 2 (VIH2) ou des combinaisons de ceux-ci.

**9.** Polynucléotide selon la revendication 8, dans lequel ladite séquence nucléotidique comprend en outre un promoteur de CMV.

**10.** Polynucléotide selon la revendication 9, dans lequel le promoteur de CMV est un promoteur de CMV de souris.

**11.** Vecteur comprenant le polynucléotide selon l'une quelconque des revendications 7 à 10.

**12.** Vecteur selon la revendication 11 pouvant être obtenu par un procédé selon l'une quelconque des revendications 1 à 6.

**13.** Vecteur selon la revendication 11 ou la revendication 12 qui est un vecteur d'expression, dans lequel les promoteurs desdits îlots CpG étendus liés exempts de méthylation sont orientés pour entraîner la transcription divergente de deux modules d'expression devant être co-exprimés.

**14.** Cellule hôte contenant un vecteur selon l'une quelconque des revendications 11 à 13.

**15.** Cellule hôte selon la revendication 14 qui contient un vecteur d'expression selon la revendication 13.

**16.** Procédé de production d'une protéine ou d'un polypeptide qui comprend la mise en culture d'une cellule hôte selon la revendication 15 dans laquelle lesdits modules d'expression sont co-exprimés et l'isolement de ladite protéine.

**17.** Procédé selon la revendication 16, dans lequel les modules d'expression transcrits de manière divergente expriment des chaînes lourdes et légères d'une immunoglobuline et ladite immunoglobuline est isolée.

**18.** Polynucléotide ou vecteur selon l'une quelconque des revendications 7 à 13 destiné à être utilisé comme médicament.

*Fig 1:* ARTIFICIAL UCOE Restriction map (Nco I fragment)

5232 bp

```
   1    CATGGCACCT  GTATTGTACT  CTTATCAGTC  ATTATATGGA  CTTTAACTTC
        GTACCGTGGA  CATAACATGA  GAATAGTCAG  TAATATACCT  GAAATTGAAG
  51    CCCAGATATT  ATTTGGGCTC  CTCCATAAGA  CTGTGAGCAT  CTGACCACTG
        GGGTCTATAA  TAAACCCGAG  GAGGTATTCT  GACACTCGTA  GACTGGTGAC
 101    GAGTGTTGCT  TCCCATTATA  TCCCTGTTAT  CAAGCACAAG  GTCAGGCACA
        CTCACAACGA  AGGGTAATAT  AGGGACAATA  GTTCGTGTTC  CAGTCCGTGT
 151    GAGTAAGACT  CAAAACATGT  TTTGGAATGT  ATGACTGGTA  TGAACTACAA
        CTCATTCTGA  GTTTTGTACA  AAACCTTACA  TACTGACCAT  ACTTGATGTT
 201    ACCAGTAAGC  TGATGTTTTC  ATTTTGAGTC  TATAAATCTA  ATTTTGTGGT
        TGGTCATTCG  ACTACAAAAG  TAAAACTCAG  ATATTTAGAT  TAAAACACCA
 251    GGTTTTGTGT  ATGGCTCAAG  GCTCAAATTG  TAAAATTTAA  TATTATGTGA
        CCAAAACACA  TACCGAGTTC  CGAGTTTAAC  ATTTTAAATT  ATAATACACT
 301    CCAAAGAAAG  TTATACCCAG  AACCTCAATT  TCCTCACCTT  CAAAATGGGG
        GGTTTCTTTC  AATATGGGTC  TTGGAGTTAA  AGGAGTGGAA  GTTTTACCCC
 351    CAGTTTCTCA  CTCATTGGTC  TGCTGTCACG  ATTTTAATGA  GCTCATGCAC
        GTCAAAGAGT  GAGTAACCAG  ACGACAGTGC  TAAAATTACT  CGAGTACGTG
 401    AAACAGCCCT  TTATATAAGG  TAAGTGCTGG  ATAAATGTTG  GCTACTATAA
        TTTGTCGGGA  AATATATTCC  ATTCACGACC  TATTTACAAC  CGATGATATT
 451    TAAAATAAGC  CTCTAAGATA  CTTGGTCAGC  ACAAGTACTA  CCCAAGAGTA
        ATTTTATTCG  GAGATTCTAT  GAACCAGTCG  TGTTCATGAT  GGGTTCTCAT
 501    TGCACTGTAA  GTAAACTGAC  AAAATTGTGT  ATCTAAAACT  GGCCAGATGA
        ACGTGACATT  CATTTGACTG  TTTTAACACA  TAGATTTTGA  CCGGTCTACT
 551    AAGAGAAACT  TTTAAGGGGC  CCTTCTGCGT  GCCCGACACT  GTGCTAGGCA
        TTCTCTTTGA  AAATTCCCCG  GGAAGACGCA  CGGGCTGTGA  CACGATCCGT
 601    CTCACACTAT  CCCGACCCGA  GAAACCGATC  TGCGACCCAG  AGGAACTTAC
        GAGTGTGATA  GGGCTGGGCT  CTTTGGCTAG  ACGCTGGGTC  TCCTTGAATG
 651    CAAGCCTCCA  GCATCTTGTG  CAGCCCTACT  CATGGGACCA  TCTGGATACC
        GTTCGGAGGT  CGTAGAACAC  GTCGGGATGA  GTACCCTGGT  AGACCTATGG
 701    CACCCTTGTC  TTTACAGGGA  GCAGAACACA  CCTCTTATGT  GTCAGAAAAC
        GTGGGAACAG  AAATGTCCCT  CGTCTTGTGT  GGAGAATACA  CAGTCTTTTG
 751    AAAGTCCAGG  AAGTATATTT  TTACCTGAGG  CAATATCTGA  AAATTGTATG
        TTTCAGGTCC  TTCATATAAA  AATGGACTCC  GTTATAGACT  TTTAACATAC
 801    CTACAGCCTC  CAAAGTGAGT  CTTCCTCTCA  GTACCTCTCT  TCTAGGCACA
        GATGTCGGAG  GTTTCACTCA  GAAGGAGAGT  CATGGAGAGA  AGATCCGTGT
 851    TGGAGCCCTT  TCTTCCAAGT  ATTATGTTTA  ACCACTTAAT  GAATGAAGTC
        ACCTCGGGAA  AGAAGGTTCA  TAATACAAAT  TGGTGAATTA  CTTACTTCAG
 901    CTGAAACTGC  TTACCCATGC  TCCCTATAAT  CTCTGAGTAA  TCTTCCTTTT
        GACTTTGACG  AATGGGTACG  AGGGATATTA  GAGACTCATT  AGAAGGAAAA
 951    CCACAACCTC  AGGCATAATC  TCATCTTCTG  TTTCTATTAC  AATTTCAAAT
        GGTGTTGGAG  TCCGTATTAG  AGTAGAAGAC  AAAGATAATG  TTAAAGTTTA
1001    TCTGGAAAAA  GGAAGTTGTG  GTCTGGAATT  ATATGGTCCA  GATGATCTGA
        AGACCTTTTT  CCTTCAACAC  CAGACCTTAA  TATACCAGGT  CTACTAGACT
1051    AACAAAAAGG  ACAGCACTAT  TAGTAATCAT  TTAGTTTTGA  AGACAGTCTA
        TTGTTTTTCC  TGTCGTGATA  ATCATTAGTA  AATCAAAACT  TCTGTCAGAT
1101    ATAATTTGCT  GTCTCTAAAG  TACTATATTC  CCTATAGTTC  TGGCATTTTA
        TATTAAACGA  CAGAGATTTC  ATGATATAAG  GGATATCAAG  ACCGTAAAAT
1151    GATAAAGGGT  CATAAATTAA  ATGCCTATAT  GGTGACATTA  TTCAGTGATT
        CTATTTCCCA  GTATTTAATT  TACGGATATA  CCACTGTAAT  AAGTCACTAA
1201    CAGACTTCAC  AGCCTTTTTT  TTTTTTTTAC  AAAGGTGTTC  CAGGCATGAA
        GTCTGAAGTG  TCGGAAAAAA  AAAAAAAATG  TTTCCACAAG  GTCCGTACTT
1251    AAATTTTAAA  GTACTATACC  TTTCCTAATT  TTACCTTTAA  AGTTGTCCTG
        TTTAAAATTT  CATGATATGG  AAAGGATTAA  AATGGAAATT  TCAACAGGAC
1301    GAAATATCTG  GGTTGACAAA  GGCGATGAAA  CTGAACTGAG  ACTTAAAAAA
        CTTTATAGAC  CCAACTGTTT  CCGCTACTTT  GACTTGACTC  TGAATTTTTT
1351    AAGATTACCC  ACCTGGTTGT  GCACAAGCCT  GCTTATGTCC  CAATCTCCAG
        TTCTAATGGG  TGGACCAACA  CGTGTTCGGA  CGAATACAGG  GTTAGAGGTC
1401    TCTAGGGTCT  GATGCTCCTT  GCTGCAGTAA  TATGCTTTGT  GGCATCTGGA
        AGATCCCAGA  CTACGAGGAA  CGACGTCATT  ATACGAAACA  CCGTAGACCT
```

*Fig 2: PDCO2/ACTIN ARTIFICIAL UCOE SEQUENCE*

```
1451   GCACGTTTTG GGGCCTAAAC AGCCACAAAC CCTGCAGAGA TGAGCACCAG
       CGTGCAAAAC CCCGGATTTG TCGGTGTTTG GGACGTCTCT ACTCGTGGTC
1501   ACTTAAGCTG GAGACACACT GATTCTCCTG TTTCTGGGGG AGGATTCTCA
       TGAATTCGAC CTCTGTGTGA CTAAGAGGAC AAAGACCCCC TCCTAAGAGT
1551   GAAGGTGGCT CATATGAGTA AAAATCGTTT TTCCTGGGTA GTTGATTCCT
       CTTCCACCGA GTATACTCAT TTTTAGCAAA AAGGACCCAT CAACTAAGGA
1601   AAAAACTAAA AAAGAATACA GAGAAAGTT TTATCTTCAA ACAAAACAGC
       TTTTTGATTT TTTCTTATGT CTCTTTTCAA AATAGAAGTT TGTTTTGTCG
1651   AATTCACATA TTTTATCCTC TGCACGTAAA ACTGAAAATA ACAACAACAA
       TTAAGTGTAT AAAATAGGAG ACGTGCATTT TGACTTTTAT TGTTGTTGTT
1701   AAAAGAAATG AAAGTTTTTG CTTTCAGGAA TAAGCTTTTA AAATCCAGAA
       TTTTCTTTAC TTTCAAAAAC GAAAGTCCTT ATTCGAAAAT TTTAGGTCTT
1751   ACTAGATTTC GTCCGGTACA CGCAACTGAG TTGCCTCCTA GAGGTGGTTT
       TGATCTAAAG CAGGCCATGT GCGTTGACTC AACGGAGGAT CTCCACCAAA
1801   GAGTTAATCA AATTAATAAG ACTGATCGTT AAGAACGACT GCCAAAAATA
       CTCAATTAGT TTAATTATTC TGACTAGCAA TTCTTGCTGA CGGTTTTTAT
1851   CGAAAAAGCT ACTGGGATCC ATCTTTCCAA GACAATTTCT ATTATCTGAA
       GCTTTTTCGA TGACCCTAGG TAGAAAGGTT CTGTTAAAGA TAATAGACTT
1901   TTAACACCAT ACCTGGTACC CACTGATTAA AAGCTGGGGG TTACCAATGC
       AATTGTGGTA TGGACCATGG GTGACTAATT TTCGACCCCC AATGGTTACG
1951   GCGTGGGCAC AGTTAGAAGC TTATGTAGCA AAAATGAGCA CATCCTGGAA
       CGCACCCGTG TCAATCTTCG AATACATCGT TTTTACTCGT GTAGGACCTT
2001   GGGCCCGGGA GAAGGTGCTC CTGGGGCAGC GCGGAGAGGG AGCTCTGAGG
       CCCGGGCCCT CTTCCACGAG GACCCCGTCG CGCCTCTCCC TCGAGACTCC
2051   CTGGGGCGGC AGCGGTGCTT GCCGCCGTCC CCCTGGTCGC TCCCGGAATT
       GACCCCGCCG TCGCCACGAA CGGCGGCAGG GGGACCAGCG AGGGCCTTAA
2101   AACGCCGCGC ACGCGTCGGA GGCATGGCCC CGTCCCGACC CCGTTTGGCG
       TTGCGGCGCG TGCGCAGCCT CCGTACCGGG GCAGGGCTGG GGCAAACCGC
2151   GCTCACCTCG CAGGCCGGCA CAGCACGGCT GCTCGCGGCA GCAGAAGAGG
       CGAGTGGAGC GTCCGGCCGT GTCGTGCCGA CGAGCGCCGT CGTCTTCTCC
2201   AAGATGCAGC GGTGGAAGGC GTCCGGGCGG CCAGGCAGCG GCGCATACAC
       TTCTACGTCG CCACCTTCCG CAGGCCCGCC GGTCCGTCGC CGCGTATGTG
2251   CTGCAGCAGG AAGGAGAGCG GGCGGCCGCA CAGCTCGCAG GCCAGGGCCT
       GACGTCGTCC TTCCTCTCGC CCGCCGGCGT GTCGAGCGTC CGGTCCCGGA
2301   GGGGCCCCGG CAGCCCGGCC GCGCCCAGCC ATGCCGGCCG CCCGCCCACC
       CCCCGGGGCC GTCGGGCCGG CGCGGGTCGG TACGGCCGGC GGGCGGGTGG
2351   TTGCTGGGGA ACTGCTCGCT GCGCAGTCGC CACGCCGGCG CCGACTCGGC
       AACGACCCCT TGACGAGCGA CGCGTCAGCG GTGCGGCCGC GGCTGAGCCG
2401   GAAGCCCAGC TCCACAGGCC TGGCCCCGGC GGCAGCCATG CGGGGCGCGG
       CTTCGGGTCG AGGTGTCCGG ACCGGGGCCG CCGTCGGTAC GCCCCGCGCC
2451   GCTGGCGTGG GGCGCAGCCC ACAGCTGGGT CGGAAGGCGG AAATCGGGCG
       CGACCGCACC CCGCGTCGGG TGTCGACCCA GCCTTCCGCC TTTAGCCCGC
2501   CCGGGCCGGA AGGCAAGAGG CGGGCACCTT TCCGGAGGAC AGGAGGCGGA
       GGCCCGGCCT TCCGTTCTCC GCCCGTGGAA AGGCCTCCTG TCCTCCGCCT
2551   AACGCGTCTG ACGGGAGCGG TTGCAGGACC AATGCGAGGG AACGGGGCAG
       TTGCGCAGAC TGCCCTCGCC AACGTCCTGG TTACGCTCCC TTGCCCCGTC
2601   AGGAAACCTC TCGGCATCAG CCCCGCCCCT GGCGCCTCTG CCTCCGAGCC
       TCCTTTGGAG AGCCGTAGTC GGGGCGGGGA CCGCGGAGAC GGAGGCTCGG
2651   GCTTTCCTGG TGCCTCCGGG TGCTCTGGGA TGGTTCTGGT CTTTGGGAGA
       CGAAAGGACC ACGGAGGCCC ACGAGACCCT ACCAAGACCA GAAACCCTCT
2701   GTGGCAGCTG GTGACGGCGC TCCGCTCACC TCTGCACATG TCTTGCTGTG
       CACCGTCGAC CACTGCCGCG AGGCGAGTGG AGACGTGTAC AGAACGACAC
2751   GGCCTGCGGG TGGCCGCCAG GGAGGCAGAG CCCTCCCGCA AACCTTCCCT
       CCGGACGCCC ACCGGCGGTC CCTCCGTCTC GGGAGGGCGT TTGGAAGGGA
2801   GCTGGTGTCC ACCTCAGGGT GTGGGAAACC TGTGCGCTGG CCGAGTGCTA
       CGACCACAGG TGGAGTCCCA CACCCTTTGG ACACGCGACC GGCTCACGAT
2851   ACCAAGAGTA GGCAGTGAAA GACAAATGAA GGTTGAACAG GTAAAGTGAG
       TGGTTCTCAT CCGTCACTTT CTGTTTACTT CCAACTTGTC CATTTCACTC
```

```
2901   GACCCTACAG CGGAAACCAA GAATCCTGTG TGCCTGAGAG TAATGAAGAA
       CTGGGATGTC GCCTTTGGTT CTTAGGACAC ACGGACTCTC ATTACTTCTT
2951   GCCTCTGCAG AAGAGTCTTT TCTGTCAGTC TTAAGGTCTC TGTTTTAATG
       CGGAGACGTC TTCTCAGAAA AGACAGTCAG AATTCCAGAG ACAAAATTAC
3001   TTAGTGCTGG CTTGCTGTAC CTGAATTCCA AGGGAGGAGT GTATAATGAG
       AATCACGACC GAACGACATG GACTTAAGGT TCCCTCCTCA CATATTACTC
3051   GCATGGCCAA CCCCCACTTC CCATCATTGC CTGAACTAGT TTTTCAGGTT
       CGTACCGGTT GGGGGTGAAG GGTAGTAACG GACTTGATCA AAAAGTCCAA
3101   AACTTCAGAA TGCCCTTGGT ACCGCGGGCC CCCTCTGTGG TCCCACGCCA
       TTGAAGTCTT ACGGGAACCA TGGCGCCCGG GGGAGACACC AGGGTGCGGT
3151   CTGATCGCTG CATGCCCACC ACCTGGGTAC ACACAGTCTG TGATTCCCGG
       GACTAGCGAC GTACGGGTGG TGGACCCATG TGTGTCAGAC ACTAAGGGCC
3201   AGCAGAACGG ACCCTGCCCA CCCGGTCTTG TGTGCTACTC AGTGGACAGA
       TCGTCTTGCC TGGGACGGGT GGGCCAGAAC ACACGATGAG TCACCTGTCT
3251   CCCAAGGCAA GAAAGGGTGA CAAGGACAGG GTCTTCCCAG GCTGGCTTTG
       GGGTTCCGTT CTTTCCCACT GTTCCTGTCC CAGAAGGGTC CGACCGAAAC
3301   AGTTCCTAGC ACCGCCCCGC CCCCAATCCT CTGTGGCACA TGGAGTCTTG
       TCAAGGATCG TGGCGGGGCG GGGGTTAGGA GACACCGTGT ACCTCAGAAC
3351   GTCCCCAGAG TCCCCCAGCG GCCTCCAGAT GGTCTGGGAG GGCAGTTCAG
       CAGGGGTCTC AGGGGGTCGC CGGAGGTCTA CCAGACCCTC CCGTCAAGTC
3401   CTGTGGCTGC GCATAGCAGA CATACAACGG ACGGTGGGCC CAGACCCAGG
       GACACCGACG CGTATCGTCT GTATGTTGCC TGCCACCCGG GTCTGGGTCC
3451   CTGTGTAGAC CCAGCCCCCC CGCCCCGCAG TGCCTAGGTC ACCCACTAAC
       GACACATCTG GGTCGGGGGG GCGGGGCGTC ACGGATCCAG TGGGTGATTG
3501   GCCCCAGGCC TGGTCTTGGC TGGGCGTGAC TGTTACCCTC AAAAGCAGGC
       CGGGGTCCGG ACCAGAACCG ACCCGCACTG ACAATGGGAG TTTTCGTCCG
3551   AGCTCCAGGG TAAAAGGTGC CCTGCCCTGT AGAGCCCACT TCCTTCCCAG
       TCGAGGTCCC ATTTTCCACG GGACGGGACA TCTCGGGTGA AGGAAGGGTC
3601   GGCTGCGGCT GGGTAGGTTT GTAGCCTTCA TCACGGGCCA CCTCCAGCCA
       CCGACGCCGA CCCATCCAAA CATCGGAAGT AGTGCCCGGT GGAGGTCGGT
3651   CTGGACCGCT GGCCCCTGCC CTGTCCTGGG GAGTGTGGTC CTGCGACTCT
       GACCTGGCGA CCGGGGACGG GACAGGACCC CTCACACCAG GACGCTGAGA
3701   AATGGCCGCA AGCCACCTGA CTCCCCCAAC ACCACACTCT ACCTCTCAAG
       TTACCGGCGT TCGGTGGACT GAGGGGGTTG TGGTGTGAGA TGGAGAGTTC
3751   CCCAGGTCTC TCCCTAGTGA CCCACCCAGC ACATTTAGCT AGCTGAGCCC
       GGGTCCAGAG AGGGATCACT GGGTGGGTCG TGTAAATCGA TCGACTCGGG
3801   CACAGCCAGA GGTCCTCAGG CCCTGCTTTC AGGGCAGTTG CTCTGAAGTC
       GTGTCGGTCT CCAGGAGTCC GGGACGAAAG TCCCGTCAAC GAGACTTCAG
3851   GGCAAGGGGG AGTGACTGCC TGGCCACTCC ATGCCCTCCA AGAGCTCCTT
       CCGTTCCCCC TCACTGACGG ACCGGTGAGG TACGGGAGGT TCTCGAGGAA
3901   CTGCAGGAGC GTACAGAACC CAGGGCCCTG GCACCCGTGC AGACCCTGGC
       GACGTCCTCG CATGTCTTGG GTCCCGGGAC CGTGGGCACG TCTGGGACCG
3951   CCACCCCACC TGGGCGCTCA GTGCCCAAGA GATGTCCACA CCTAGGATGT
       GGTGGGGTGG ACCCGCGAGT CACGGGTTCT CTACAGGTGT GGATCCTACA
4001   CCCGCGGTGG GTGGGGGGCC CGAGAGACGG GCAGGCCGGG GGCAGGCCTG
       GGGCGCCACC CACCCCCCGG GCTCTCTGCC CGTCCGGCCC CCGTCCGGAC
4051   GCCATGCGGG GCCGAACCGG GCACTGCCCA GCGTGGGGCG CGGGGGCCAC
       CGGTACGCCC CGGCTTGGCC CGTGACGGGT CGCACCCCGC GCCCCCGGTG
4101   GGCGCGCGCC CCCAGCCCCC GGGCCCAGCA CCCCAAGGCG GCCAACGCCA
       CCGCGCGCGG GGGTCGGGGG CCCGGGTCGT GGGGTTCCGC CGGTTGCGGT
4151   AAACTCTCCC TCCTCCTCTT CCTCAATCTC GCTCTCGCTC TTTTTTTTTT
       TTTGAGAGGG AGGAGGAGAA GGAGTTAGAG CGAGAGCGAG AAAAAAAAAA
4201   TCGCAAAAGG AGGGGAGAGG GGGTAAAAAA ATGCTGCACT GTGCGGCGAA
       AGCGTTTTCC TCCCCTCTCC CCCATTTTTT TACGACGTGA CACGCCGCTT
4251   GCCGGTGAGT GAGCGGCGCG GGGCCAATCA GCGTGCGCCG TTCCGAAAGT
       CGGCCACTCA CTCGCCGCGC CCCGGTTAGT CGCACGCGGC AAGGCTTTCA
4301   TGCCTTTTAT GGCTCGAGCG GCCGCGGCGG CGCCCTATAA AACCCAGCGG
       ACGGAAAATA CCGAGCTCGC CGGCGCCGCC GCGGGATATT TTGGGTCGCC
```

```
4351   CGCGACGCGC  CACCACCGCC  GAGACCGCGT  CCGCCCGCGA  GCACAGAGCC
       GCGCTGCGCG  GTGGTGGCGG  CTCTGGCGCA  GGCGGGCGCT  CGTGTCTCGG
4401   TCGCCTTTGC  CGATCCGCCG  CCCGTCCACA  CCCGCCGCCA  GGTAAGCCCG
       AGCGGAAACG  GCTAGGCGGC  GGGCAGGTGT  GGGCGGCGGT  CCATTCGGGC
4451   GCCAGCCGAC  CGGGGCATGC  GGCCGCGGCC  CTTCGCCCGT  GCAGAGCCGC
       CGGTCGGCTG  GCCCCGTACG  CCGGCGCCGG  GAAGCGGGCA  CGTCTCGGCG
4501   CGTCTGGGCC  GCAGCGGGGG  GCGCATGGGG  CGGAACCGGA  CCGCCGTGGG
       GCAGACCCGG  CGTCGCCCCC  CGCGTACCCC  GCCTTGGCCT  GGCGGCACCC
4551   GGGCGCGGGA  GAAGCCCCTG  GGCCTCCGGA  GATGGGGGAC  ACCCCACGCC
       CCCGCGCCCT  CTTCGGGGAC  CCGGAGGCCT  CTACCCCCTG  TGGGGTGCGG
4601   AGTTCGCAGG  CGCGAGGCCG  CGCTCGGGCG  GGCGCGCTCC  GGGGGTGCCG
       TCAAGCGTCC  GCGCTCCGGC  GCGAGCCCGC  CCGCGCGAGG  CCCCCACGGC
4651   CTCTCGGGGC  GGGGGCAACC  GGCGGGGTCT  TTGTCTGAGC  CGGGCTCTTG
       GAGAGCCCCG  CCCCCGTTGG  CCGCCCCAGA  AACAGACTCG  GCCCGAGAAC
4701   CCAATGGGGA  TCGCACGGTG  GGCGCGGCGT  AGCCCCCGTC  AGGCCCGGTG
       GGTTACCCCT  AGCGTGCCAC  CCGCGCCGCA  TCGGGGGCAG  TCCGGGCCAC
4751   GGGGCTGGGG  CGCCATGCGC  GTGCGCGCTG  GTCCTTTGGG  CGCTAACTGC
       CCCCGACCCC  GCGGTACGCG  CACGCGCGAC  CAGGAAACCC  GCGATTGACG
4801   GTGCGCGCTG  GGAATTGGCG  CTAATTGCGC  GTGCGCGCTG  GGACTCAATG
       CACGCGCGAC  CCTTAACCGC  GATTAACGCG  CACGCGCGAC  CCTGAGTTAC
4851   GCGCTAATCG  CGCGTGCGTT  CTGGGGCCCG  GGCGCTTGCG  CCACTTCCTG
       CGCGATTAGC  GCGCACGCAA  GACCCCGGGC  CCGCGAACGC  GGTGAAGGAC
4901   CCCGAGCCGC  TGGCGCCCGA  GGGTGTGGCC  GCTGCGTGCG  CGCGCGCGAC
       GGGCTCGGCG  ACCGCGGGCT  CCCACACCGG  CGACGCACGC  GCGCGCGCTG
4951   CCGGTCGCTG  TTTGAACCGG  GCGGAGGCGG  GGCTGGCGCC  CGGTTGGGAG
       GGCCAGCGAC  AAACTTGGCC  CGCCTCCGCC  CCGACCGCGG  GCCAACCCTC
5001   GGGGTTGGGG  CCTGGCTTCC  TGCCGCGCGC  CGCGGGGACG  CCTCCGACCA
       CCCCAACCCC  GGACCGAAGG  ACGGCGCGCG  GCGCCCCTGC  GGAGGCTGGT
5051   GTGTTTGCCT  TTTATGGTAA  TAACGCGGCC  GGCCCGGCTT  CCTTTGTCCC
       CACAAACGGA  AAATACCATT  ATTGCGCCGG  CCGGGCCGAA  GGAAACAGGG
5101   CAATCTGGGC  GCGCGCCGGC  GCCCCCTGGC  GGCCTAAGGA  CTCGGCGCGC
       GTTAGACCCG  CGCGCGGCCG  CGGGGGACCG  CCGGATTCCT  GAGCCGCGCG
5151   CGGAAGTGGC  CAGGGCGGGG  GCGACTTCGG  CTCACAGCGC  GCCCGGCTAT
       GCCTTCACCG  GTCCCGCCCC  CGCTGAAGCC  GAGTGTCGCG  CGGGCCGATA
5201   TCTCGCAGCT  CACCATGCCG  GTCGCCACCA  TG
       AGAGCGTCGA  GTGGTACGGC  CAGCGGTGGT  AC
```

FIGURE 3a:

**Kan/Neo**

**PDCD2 MFI**

**CET 500**
9245 bp

*Hin* dIII (1742)

*Kpn* I (1929)

*Hin* dIII (1977)

**SV40pA**

*Kpn* I (5882)

*Sal* I (5872)

*Hin* dIII (5855)

**CMV**

*Kpn* I (3132)

**Actin MFI**

EP 1 430 124 B1

FIGURE 3b:

ACTIN MFI

Kan/Neo

CET 501
9245 bp

*Kpn* I (2124)

SV40pA

*Kpn* I (5882)

*Sal* I (5872)

*Hin* dIII (5855)

CMV

*Hin* dIII (3271)

*Kpn* I (3327)

*Hin* dIII (3506)

PDCD2 MFI

EP 1 430 124 B1

# Figure 4
## CET220 vs PDCD2/actin artificial UCOE
## Median Fluorescence

EP 1 430 124 B1

# Figure 5
## CET220 vs PDCD2/actin artificial UCOE
## % Positive Cells

EP 1 430 124 B1

# RNP/HP-1/actin artificial UCOE restriction map

**(Please note that this figure is complementary to the sequence of Figure 7)**

*Sac*I (1352)

*Not*I (921)

*Not*I (770)                     *Bsp*EI (3649)

*Bsp*EI (665)                              *Sac*I (5996)

*Sal*I (2)         *Kpn*I (2126)          *Not*I (4722)       *Hind*III (6299

**ACTIN MFI**              **Hybid UCOE**        **RNP/HP-1 MFI**

6303 bp

*Figure 6:*

```
   1   AAGCTTACTT GATTGGCCAT GTGGCAAGCG ACAGGCACAA AACAATTTTC
       TTCGAATGAA CTAACCGGTA CACCGTTCGC TGTCCGTGTT TTGTTAAAAG
  51   CAAGTCAATA GGAAAAACCT CAGAGCTGAA ATCTTTATAT GCTGTACTAC
       GTTCAGTTAT CCTTTTTGGA GTCTCGACTT TAGAAATATA CGACATGATG
 101   ACAGCTGTAT TCTGGGCACT TATGAATGTT AAGGAAACCT GTCTTAAAAG
       TGTCGACATA AGACCCGTGA ATACTTACAA TTCCTTTGGA CAGAATTTTC
 151   TTAACTAGGT TAAAAAACCT CAAACGAGAG AAAGTGATAT CCAGGACCAA
       AATTGATCCA ATTTTTTGGA GTTTGCTCTC TTTCACTATA GGTCCTGGTT
 201   CTGCTACAAA CGCATAATGC AAACTAAAAA GTCACACGTA ATTTTCAATC
       GACGATGTTT GCGTATTACG TTTGATTTTT CAGTGTGCAT TAAAAGTTAG
 251   AATTATTTTT TGTTCCTAGC AAGCAGCATT AATTGCTGCT CTCATCCCAG
       TTAATAAAAA ACAAGGATCG TTCGTCGTAA TTAACGACGA GAGTAGGGTC
 301   TTCTACGGAG CTCTCCCTCC ATTCGCATGC TCCCAACTCC TAAAAAGTAG
       AAGATGCCTC GAGAGGGAGG TAAGCGTACG AGGGTTGAGG ATTTTTCATC
 351   TGGTAAAACC CAGTTCAGAT TTTTTTTCCT GTAGTTTTCA TGACTCGTAA
       ACCATTTTGG GTCAAGTCTA AAAAAAAGGA CATCAAAAGT ACTGAGCATT
 401   AAATTAAAGA AAAAATTAAC TGAAATGATC AAACTAGCTC CTATGAGACA
       TTTAATTTCT TTTTTAATTG ACTTTACTAG TTTGATCGAG GATACTCTGT
 451   CAAAGCAGTC TTTTGAAATG GTTACTTGTC ACGATAGTTA TTTTCATTTT
       GTTTCGTCAG AAAACTTTAC CAATGAACAG TGCTATCAAT AAAAGTAAAA
 501   TTCAGCTAGT TTTTATTCTT AATTGTCGTC AGCACATAGG TTATCTCTAA
       AAGTCGATCA AAAATAAGAA TTAACAGCAG TCGTGTATCC AATAGAGATT
 551   ACTGAAATTA CGGATAATGT ACATTATAA CAAGTTTTAC AAATCACTAA
       TGACTTTAAT GCCTATTACA TGTAAATATT GTTCAAAATG TTTAGTGATT
 601   CAAAAAGCAA AAACTCATTA CTTACCTCAC AATTTATCCA AACTTACCCG
       GTTTTTCGTT TTTGAGTAAT GAATGGAGTG TTAAATAGGT TTGAATGGGC
 651   ATGTCCACTA TCGATTTTAA ACAATGTTAT TTTATAAACG TGCTTAGGGT
       TACAGGTGAT AGCTAAAATT TGTTACAATA AAATATTTGC ACGAATCCCA
 701   CAAAGAAAAA TAACCAGGTA GACCCCCTTC GCTTGAGACC TTATGCTTAT
       GTTTCTTTTT ATTGGTCCAT CTGGGGGAAG CGAACTCTGG AATACGAATA
 751   CAATGTAATG TTCAACCAAG ATTGCAAACA AAATGAGAAA AGTAACAAAG
       GTTACATTAC AAGTTGGTTC TAACGTTTGT TTTACTCTTT TCATTGTTTC
 801   TTCAAATACA GAGCGGCCCA GGCCCAAAAC AGTTTTGCAC ATCAATCCAT
       AAGTTTATGT CTCGCCGGGT CCGGGTTTTG TCAAAACGTG TAGTTAGGTA
 851   ACGCATTACA GGAAGGAGCC TCTGAAGCCA TGTTTTAATC GAAGTATAAC
       TGCGTAATGT CCTTCCTCGG AGACTTCGGT ACAAAATTAG CTTCATATTG
 901   TAAGGACAAA ATCGTTATTT CACTTTCCTC GTAATCATCT ATAAAGGTCC
       ATTCCTGTTT TAGCAATAAA GTGAAAGGAG CATTAGTAGA TATTTCCAGG
 951   ATGGATCTGT CCCGTAAGGG TTAAACTTCT CAGTAACAAC ATTACTTAAA
       TACCTAGACA GGGCATTCCC AATTTGAAGA GTCATTGTTG TAATGAATTT
1001   ATGAGTCAGC TCTACAACTT AAACGGAATC CTTAAGAACA GTAAAGGATT
       TACTCAGTCG AGATGTTGAA TTTGCCTTAG GAATTCTTGT CATTTCCTAA
1051   CTGACGCGAA TATCCCTCCC CCGCCCAGAA AACCACCTTC GTCCCTGCCC
       GACTGCGCTT ATAGGGAGGG GGCGGGTCTT TTGGTGGAAG CAGGGACGGG
1101   CTCGTGGCCG ATGGCTTCCA ATTTATGTTT ATTTTGCCGC GGTTCATCTG
       GAGCACCGGC TACCGAAGGT TAAATACAAA TAAAACGGCG CCAAGTAGAC
1151   TCGTTTTACT GACTGCAGAC CCAGATAAAA CCGTTACTCA AAGGAAAAAA
       AGCAAAATGA CTGACGTCTG GGTCTATTTT GGCAATGAGT TTCCTTTTTT
1201   AAGACAGGAA AAACATAAAA TGGTTTCTTT GTCCTACGGC TCGCATTGAA
       TTCTGTCCTT TTTGTATTTT ACCAAAGAAA CAGGATGCCG AGCGTAACTT
1251   CCCGGCCCGA CGCCCTGGGT GGTGATATCT TCTCTGAAAC CGGGCCCGCA
       GGGCCGGGCT GCGGGACCCA CCACTATAGA AGAGACTTTG GCCCGGGCGT
1301   AACCCGGAGC ACCCCCCCTC CCCGCTCTTC GGTGTGGCTT CCGAACGCAA
       TTGGGCCTCG TGGGGGGGAG GGGCGAGAAG CCACACCGAA GGCTTGCGTT
1351   TGGCGCCATT TCATCGAGGG GAAGGCTGAG CGCCTTTAAT GAGGTGCGCA
       ACCGCGGTAA AGTAGCTCCC CTTCCGACTC GCGGAAATTA CTCCACGCGT
1401   GGACTCTAAA GATCCAAGCT CACAAAACAC TCCAAATCCA CCTCGAAACG
       CCTGAGATTT CTAGGTTCGA GTGTTTTGTG AGGTTTAGGT GGAGCTTTGC
```

*Figure 7: RNP/HP-1/Actin Artificial UCOE Sequence.*

```
1451  ATATGAAAAC AGCCCGAGAA GAAAAAAAAA ATAGTTAACC ACTTCTACTT
      TATACTTTTG TCGGGCTCTT CTTTTTTTTT TATCAATTGG TGAAGATGAA
1501  CTTGATAGAG AAAGCACACT AAGAAAATAA AAGAGTTATA AGGAAAACGC
      GAACTATCTC TTTCGTGTGA TTCTTTTATT TTCTCAATAT TCCTTTTGCG
1551  TGAGAGGAAG GCGAGCCATG AAAATGGCGG CCGCCAAATC GGTTCCCGGG
      ACTCTCCTTC CGCTCGGTAC TTTTACCGCC GGCGGTTTAG CCAAGGGCCC
1601  AGAGAGGGGG AGGGGAAGCT CCGCAGCCTC GCTCACGAGG ACCTGCTGCC
      TCTCTCCCCC TCCCCTTCGA GGCGTCGGAG CGAGTGCTCC TGGACGACGG
1651  CGCCGAAACG CTCGCCGAGG AGACGCCGTG GCCCCCGAAG CAGCGTGCTT
      GCGGCTTTGC GAGCGGCTCC TCTGCGGCAC CGGGGGCTTC GTCGCACGAA
1701  TAGAAAGGGA ATAAGAATTC CCGCCTCCGC GCCCCACTTT CACCCCAGCG
      ATCTTTCCCT TATTCTTAAG GGCGGAGGCG CGGGGTGAAA GTGGGGTCGC
1751  GGGCAGCGTC CGCCATGTGA AAGCTCCCCA TCCCCCACCC CCAGTGAAGG
      CCCGTCGCAG GCGGTACACT TTCGAGGGGT AGGGGGTGGG GGTCACTTCC
1801  GAAATGGCGC CGGGAGGCTG AGGGTGGGGA AGCTGTTTGT ACGCTCAGGC
      CTTTACCGCG GCCCTCCGAC TCCCACCCCT TCGACAAACA TGCGAGTCCG
1851  CTCCGCTCAA GACCCCGTTC ATAAACCTTA AGCCCCACTG CTACTGAATT
      GAGGCGAGTT CTGGGGCAAG TATTTGGAAT TCGGGGTGAC GATGACTTAA
1901  GGTCCGATTT CCTGCCTCTC TCCCACGGAG GCGGCTGGCC GACTTCCACT
      CCAGGCTAAA GGACGGAGAG AGGGTGCCTC CGCCGACCGG CTGAAGGTGA
1951  GAGGCGCCAA CGGCCTCGCC ATGCCCTTTT CAATAACTCA TTGATTTCAA
      CTCCGCGGTT GCCGGAGCGG TACGGGAAAA GTTATTGAGT AACTAAAGTT
2001  ACCCGTTACC TCCATCGCGG ACTCAGTCGC TTCAGCCCGA TTTCCCGCAG
      TGGGCAATGG AGGTAGCGCC TGAGTCAGCG AAGTCGGGCT AAAGGGCGTC
2051  CCGAGCGAGA TGAGAGAGAT CTCCGCGGAC GAACACGAAC CGGACTCGTC
      GGCTCGCTCT ACTCTCTCTA GAGGCGCCTG CTTGTGCTTG GCCTGAGCAG
2101  CTGGCGCTGT AGTGAGAACT GCCGCTGCTC GAGAAACAAC TCTGCGAGGA
      GACCGCGACA TCACTCTTGA CGGCGACGAG CTCTTTGTTG AGACGCTCCT
2151  GCACCTCCGC ACGGGACCCG GCGCTGCTGC TACTGCCGCT AGAGCCGCTG
      CGTGGAGGCG TGCCCTGGGC CGCGACGACG ATGACGGCGA TCTCGGCGAC
2201  CCGCCGCTTT TCTAGAACCT TCCCCCCCAC TAACGCGTCT TCCGCTACGT
      GGCGGCGAAA AGATCTTGGA AGGGGGGGTG ATTGCGCAGA AGGCGATGCA
2251  CAGGCCGTCG CGTAAACGCC CTATCCGCCG CCAATGGCGG GAAGGCTCTA
      GTCCGGCAGC GCATTTGCGG GATAGGCGGC GGTTACCGCC CTTCCGAGAT
2301  CGCCCCACCT TACGCCAAAT GCGTACTCCT CCCACCCTTG CGGCCAGAGA
      GCGGGGTGGA ATGCGGTTTA CGCATGAGGA GGGTGGGAAC GCCGGTCTCT
2351  CAGTACCCGA CGTTACTTCC GTAAATGCGC TCAATGAATT GCGGAAGGCT
      GTCATGGGCT GCAATGAAGG CATTTACGCG AGTTACTTAA CGCCTTCCGA
2401  AGAGTCCTGC TAGTTACTAC CTCTTGGAAT AGGGTCCCGG CCCCTGCCTT
      TCTCAGGACG ATCAATGATG GAGAACCTTA TCCCAGGGCC GGGGACGGAA
2451  GGCGAAGGCA GGTGAGAAAC GTCGCGCAGT TTGAAATTAA CGCCGACGGG
      CCGCTTCCGT CCACTCTTTG CAGCGCGTCA AACTTTAATT GCGGCTGCCC
2501  AGGGGCTTAA TCCGCAGCCT GGAGATCCAG CCCCCTCAAC CCGGGAGGTG
      TCCCCGAATT AGGCGTCGGA CCTCTAGGTC GGGGGAGTTG GGCCCTCCAC
2551  GTCCCTGCAG TTACGCCAAT GATAACCCCC GCCAGAAAAA TCTTAGTAGC
      CAGGGACGTC AATGCGGTTA CTATTGGGGG CGGTCTTTTT AGAATCATCG
2601  CTTCCCTTTT TGTTTTCCGT GCCCCAACTC GGCGGATTGA CTCGGCCCCT
      GAAGGGAAAA ACAAAAGGCA CGGGGTTGAG CCGCCTAACT GAGCCGGGGA
2651  TCCGGAAACA CCCGAATCAA CTTCTAGTCA AATTATTGTT CACGCCGCAA
      AGGCCTTTGT GGGCTTAGTT GAAGATCAGT TTAATAACAA GTGCGGCGTT
2701  TGACCCACCC CTGGCCCGCG TCTGTGGAAC TGACCCCTGG TGTACAGGAG
      ACTGGGTGGG GACCGGGCGC AGACACCTTG ACTGGGGACC ACATGTCCTC
2751  AGTTCGCTGC TGAAAGTGGT CCCAAAGGGG TACTAGTTTT TAAGCTCCCA
      TCAAGCGACG ACTTTCACCA GGGTTTCCCC ATGATCAAAA ATTCGAGGGT
2801  ACTCCCCCTC CCCCAGCGTC TGGAGGATTC CACACCCTCG CACCGGCGGG
      TGAGGGGGAG GGGGTCGCAG ACCTCCTAAG GTGTGGGAGC GTGGCCGCCC
2851  CGAGGAAGTG GGCGGAGTCC GGTTTTGGCG CCAGCCGCTG AGGCTGCCAA
      GCTCCTTCAC CCGCCTCAGG CCAAAACCGC GGTCGGCGAC TCCGACGGTT
```

```
2901   GCAGAAAAGC  CACCGCTGAG  GAGACTCCGG  TCACTGTCCT  CGCCCCGCCT
       CGTCTTTTCG  GTGGCGACTC  CTCTGAGGCC  AGTGACAGGA  GCGGGGCGGA
2951   CCCCCTTCCC  TCCCCTTGGG  GACCACCGGG  CGCCACGCCG  CGAACGGTAA
       GGGGGAAGGG  AGGGGAACCC  CTGGTGGCCC  GCGGTGCGGC  GCTTGCCATT
3001   GTGCCGCGGT  CGTCGGCGCC  TCCGCCCTCC  CCCTAGGGCC  CCAATTCCCA
       CACGGCGCCA  GCAGCCGCGG  AGGCGGGAGG  GGGATCCCGG  GGTTAAGGGT
3051   GCGGGCGCGG  CGCCGGCCCC  TCCCCCCGCC  GCGCGCGCGC  CCGCTGCCCC
       CGCCCGCGCC  GCGGCCGGGG  AGGGGGGCGG  CGCGCGCGCG  GGCGACGGGG
3101   GCCCTTCGTG  GCCGCCCGGC  GTGGGCGGTG  CCACCCCTCC  CCCCGGCGGC
       CGGGAAGCAC  CGGCGGGCCG  CACCCGCCAC  GGTGGGGAGG  GGGGCCGCCG
3151   CCCGCGCGCA  GCTCCCGGCT  CCCTCCCCCT  TCGGATGTGG  CTTGAGCTGT
       GGGCGCGCGT  CGAGGGCCGA  GGGAGGGGGA  AGCCTACACC  GAACTCGACA
3201   AGGCGCGGAG  GGCCGGAGAC  GCTGCAGACC  CGCGACCCGG  AGCAGCTCGG
       TCCGCGCCTC  CCGGCCTCTG  CGACGTCTGG  GCGCTGGGCC  TCGTCGAGCC
3251   AGGCGGTGAA  GTCGGTGGCT  TTCCTTCTCT  CTAGCTCTCG  CTCGCTGGTG
       TCCGCCACTT  CAGCCACCGA  AAGGAAGAGA  GATCGAGAGC  GAGCGACCAC
3301   GTGCTTCAGA  TGCCACACGC  GTCCCGGGGG  CCCGGTTCTC  CGCTCCCCTC
       CACGAAGTCT  ACGGTGTGCG  CAGGGCCCCC  GGGCCAAGAG  GCGAGGGGAG
3351   CCCTCCCCTT  CTCGCCGGAC  CCCGCGCCGG  GAGCTGCGGG  AAGGAGTGGA
       GGGAGGGGAA  GAGCGGCCTG  GGGCGCGGCC  CTCGACGCCC  TTCCTCACCT
3401   GGGTCGGGCG  GTGGCCTCGC  GGCTGGCCTG  GCGCGCGGCC  AGCCCGGTAG
       CCCAGCCCGC  CACCGGAGCG  CCGACCGGAC  CGCGCGCCGG  TCGGGCCATC
3451   TTAGTGGGGG  GACTGCTCTG  CCCTCGAGGG  GGTAGGGAGC  TGTGGCGACG
       AATCACCCCC  CTGACGAGAC  GGGAGCTCCC  CCATCCCTCG  ACACCGCTGC
3501   GTTGCCCCAT  TTCGAGACAA  AGCGCATTTC  CCCCTCCCCT  CCCCCACCCG
       CAACGGGGTA  AAGCTCTGTT  TCGCGTAAAG  GGGGAGGGGA  GGGGGTGGGC
3551   CGTTCCGGCG  GAGGCGCCCC  CTCCCCCAGC  GCCACGCGGG  GCTGGGTCGA
       GCAAGGCCGC  CTCCGCGGGG  GAGGGGGTCG  CGGTGCGCCC  CGACCCAGCT
3601   GACTTGGGCC  TCCCGGAGGG  CGGCGCGTGG  TCCCGCGTCC  GCGAGCCTGG
       CTGAACCCGG  AGGGCCTCCC  GCCGCGCACC  AGGGCGCAGG  CGCTCGGACC
3651   CGGCGCGCGG  CCGGCTGTCC  CGAGGCTGCG  GCGACCGCCA  GTTAACGTGG
       GCCGCGCGCC  GGCCGACAGG  GCTCCGACGC  CGCTGGCGGT  CAATTGCACC
3701   CCGCGCGGGG  GTAGGCGCGT  GCGGTGTGGC  GCAGTGCCCT  TGAGCCCCCG
       GGCGCGCCCC  CATCCGCGCA  CGCCACACCG  CGTCACGGGA  ACTCGGGGGC
3751   TGCCGCGGCC  TTTGTTTCTC  CCCGCGGATG  CGCTGACCAC  GAGGCCCGCG
       ACGGCGCCGG  AAACAAAGAG  GGGCGCCTAC  GCGACTGGTG  CTCCGGGCGC
3801   CTCCGGGTG  GGGGCGGGCA  CCCGCGCTTA  GGCCTCTGGA  CGCCGGGCTT
       GAGGGCCCAC  CCCCGCCCGT  GGGCGCGAAT  CCGGAGACCT  GCGGCCCGAA
3851   CAGCGGCGGG  GGTCGGGAGC  GGGTGTTTGC  AAGAGGTGAT  TCTTTTTTCA
       GTCGCCGCCC  CCAGCCCTCG  CCCACAAACG  TTCTCCACTA  AGAAAAAAGT
3901   AAGTGTCACG  AAACGGGGTT  GAAGCATCTT  AAGTTTTTTC  CTTTTGTTAT
       TTCACAGTGC  TTTGCCCCAA  CTTCGTAGAA  TTCAAAAAAG  GAAAACAATA
3951   TTAATTACCG  ATTGGAAAGA  GGGAGGGTTT  CTGAGCAGAA  ACCAAGTTGG
       AATTAATGGC  TAACCTTTCT  CCCTCCCAAA  GACTCGTCTT  TGGTTCAACC
4001   GATTGCAGAA  CAGAGAAGAT  TCACAGTGCT  TTACCGTTGT  GAGTTGTTTG
       CTAACGTCTT  GTCTCTTCTA  AGTGTCACGA  AATGGCAACA  CTCAACAAAC
4051   GGTAATCGTG  CCTGGTTTTA  AACCGAAAGG  ATTGTCCTTT  AAAAATGGAA
       CCATTAGCAC  GGACCAAAAT  TTGGCTTTCC  TAACAGGAAA  TTTTTACCTT
4101   CATGGACTTT  ATTATAAATG  GGACTTAGAT  TGGAAAGAC  ATTGGTCCCC
       GTACCTGAAA  TAATATTTAC  CCTGAATCTA  ACCTTTTCTG  TAACCAGGGG
4151   TATTTTAAGC  CATGTGAAGC  TGTTTTAGGT  ACCGCGGGCC  CCCTCTGTGG
       ATAAAATTCG  GTACACTTCG  ACAAAATCCA  TGGCGCCCGG  GGGAGACACC
4201   TCCCACGCCA  CTGATCGCTG  CATGCCCACC  ACCTGGGTAC  ACACAGTCTG
       AGGGTGCGGT  GACTAGCGAC  GTACGGGTGG  TGGACCCATG  TGTGTCAGAC
4251   TGATTCCCGG  AGCAGAACGG  ACCCTGCCCA  CCCGGTCTTG  TGTGCTACTC
       ACTAAGGGCC  TCGTCTTGCC  TGGGACGGGT  GGGCCAGAAC  ACACGATGAG
4301   AGTGGACAGA  CCCAAGGCAA  GAAAGGGTGA  CAAGGACAGG  GTCTTCCCAG
       TCACCTGTCT  GGGTTCCGTT  CTTTCCCACT  GTTCCTGTCC  CAGAAGGGTC
```

```
4351   GCTGGCTTTG AGTTCCTAGC ACCGCCCCGC CCCCAATCCT CTGTGGCACA
       CGACCGAAAC TCAAGGATCG TGGCGGGGCG GGGGTTAGGA GACACCGTGT
4401   TGGAGTCTTG GTCCCCAGAG TCCCCCAGCG GCCTCCAGAT GGTCTGGGAG
       ACCTCAGAAC CAGGGGTCTC AGGGGGTCGC CGGAGGTCTA CCAGACCCTC
4451   GGCAGTTCAG CTGTGGCTGC GCATAGCAGA CATACAACGG ACGGTGGGCC
       CCGTCAAGTC GACACCGACG CGTATCGTCT GTATGTTGCC TGCCACCCGG
4501   CAGACCCAGG CTGTGTAGAC CCAGCCCCCC CGCCCCGCAG TGCCTAGGTC
       GTCTGGGTCC GACACATCTG GGTCGGGGGG GCGGGGCGTC ACGGATCCAG
4551   ACCCACTAAC GCCCCAGGCC TGGTCTTGGC TGGGCGTGAC TGTTACCCTC
       TGGGTGATTG CGGGGTCCGG ACCAGAACCG ACCCGCACTG ACAATGGGAG
4601   AAAAGCAGGC AGCTCCAGGG TAAAAGGTGC CCTGCCCTGT AGAGCCCACT
       TTTTCGTCCG TCGAGGTCCC ATTTTCCACG GGACGGGACA TCTCGGGTGA
4651   TCCTTCCCAG GGCTGCGGCT GGGTAGGTTT GTAGCCTTCA TCACGGGCCA
       AGGAAGGGTC CCGACGCCGA CCCATCCAAA CATCGGAAGT AGTGCCCGGT
4701   CCTCCAGCCA CTGGACCGCT GGCCCCTGCC CTGTCCTGGG GAGTGTGGTC
       GGAGGTCGGT GACCTGGCGA CCGGGGACGG GACAGGACCC CTCACACCAG
4751   CTGCGACTCT AATGGCCGCA AGCCACCTGA CTCCCCCAAC ACCACACTCT
       GACGCTGAGA TTACCGGCGT TCGGTGGACT GAGGGGGTTG TGGTGTGAGA
4801   ACCTCTCAAG CCCAGGTCTC TCCCTAGTGA CCCACCCAGC ACATTTAGCT
       TGGAGAGTTC GGGTCCAGAG AGGGATCACT GGGTGGGTCG TGTAAATCGA
4851   AGCTGAGCCC CACAGCCAGA GGTCCTCAGG CCCTGCTTTC AGGGCAGTTG
       TCGACTCGGG GTGTCGGTCT CCAGGAGTCC GGGACGAAAG TCCCGTCAAC
4901   CTCTGAAGTC GGCAAGGGGG AGTGACTGCC TGGCCACTCC ATGCCCTCCA
       GAGACTTCAG CCGTTCCCCC TCACTGACGG ACCGGTGAGG TACGGGAGGT
4951   AGAGCTCCTT CTGCAGGAGC GTACAGAACC CAGGGCCCTG GCACCCGTGC
       TCTCGAGGAA GACGTCCTCG CATGTCTTGG GTCCCGGGAC CGTGGGCACG
5001   AGACCCTGGC CCACCCCACC TGGGCGCTCA GTGCCCAAGA GATGTCCACA
       TCTGGGACCG GGTGGGGTGG ACCCGCGAGT CACGGGTTCT CTACAGGTGT
5051   CCTAGGATGT CCCGCGGTGG GTGGGGGGCC CGAGAGACGG GCAGGCCGGG
       GGATCCTACA GGGCGCCACC CACCCCCCGG GCTCTCTGCC CGTCCGGCCC
5101   GGCAGGCCTG GCCATGCGGG GCCGAACCGG GCACTGCCCA GCGTGGGGCG
       CCGTCCGGAC CGGTACGCCC CGGCTTGGCC CGTGACGGGT CGCACCCCGC
5151   CGGGGGCCAC GGCGCGCGCC CCCAGCCCCC GGGCCCAGCA CCCCAAGGCG
       GCCCCCGGTG CCGCGCGCGG GGGTCGGGGG CCCGGGTCGT GGGGTTCCGC
5201   GCCAACGCCA AAACTCTCCC TCCTCCTCTT CCTCAATCTC GCTCTCGCTC
       CGGTTGCGGT TTTGAGAGGG AGGAGGAGAA GGAGTTAGAG CGAGAGCGAG
5251   TTTTTTTTTT TCGCAAAAGG AGGGGAGAGG GGGTAAAAAA ATGCTGCACT
       AAAAAAAAAA AGCGTTTTCC TCCCCTCTCC CCCATTTTTT TACGACGTGA
5301   GTGCGGCGAA GCCGGTGAGT GAGCGGCGCG GGGCCAATCA GCGTGCGCCG
       CACGCCGCTT CGGCCACTCA CTCGCCGCGC CCCGGTTAGT CGCACGCGGC
5351   TTCCGAAAGT TGCCTTTTAT GGCTCGAGCG GCCGCGGCGG CGCCCTATAA
       AAGGCTTTCA ACGGAAAATA CCGAGCTCGC CGGCGCCGCC GCGGGATATT
5401   AACCCAGCGG CGCGACGCGC CACCACCGCC GAGACCGCGT CCGCCCGCGA
       TTGGGTCGCC GCGCTGCGCG GTGGTGGCGG CTCTGGCGCA GGCGGGCGCT
5451   GCACAGAGCC TCGCCTTTGC CGATCCGCCG CCCGTCCACA CCCGCCGCCA
       CGTGTCTCGG AGCGGAAACG GCTAGGCGGC GGGCAGGTGT GGGCGGCGGT
5501   GGTAAGCCCG GCCAGCCGAC CGGGGCATGC GGCCGCGGCC CTTCGCCCGT
       CCATTCGGGC CGGTCGGCTG GCCCCGTACG CCGGCGCCGG GAAGCGGGCA
5551   GCAGAGCCGC CGTCTGGGCC GCAGCGGGGG GCGCATGGGG CGGAACCGGA
       CGTCTCGGCG GCAGACCCGG CGTCGCCCCC CGCGTACCCC GCCTTGGCCT
5601   CCGCCGTGGG GGGCGCGGGA GAAGCCCCTG GGCCTCCGGA GATGGGGGAC
       GGCGGCACCC CCCGCGCCCT CTTCGGGGAC CCGGAGGCCT CTACCCCCTG
5651   ACCCCACGCC AGTTCGCAGG CGCGAGGCCG CGCTCGGGCG GGCGCGCTCC
       TGGGGTGCGG TCAAGCGTCC GCGCTCCGGC GCGAGCCCGC CGCGCGAGG
5701   GGGGGTGCCG CTCTCGGGGC GGGGGCAACC GGCGGGGTCT TTGTCTGAGC
       CCCCCACGGC GAGAGCCCCG CCCCCGTTGG CCGCCCCAGA AACAGACTCG
5751   CGGGCTCTTG CCAATGGGGA TCGCACGGTG GGCGCGGCGT AGCCCCCGTC
       GCCCGAGAAC GGTTACCCCT AGCGTGCCAC CCGCGCCGCA TCGGGGGCAG
```

31

```
5801   AGGCCCGGTG GGGGCTGGGG CGCCATGCGC GTGCGCGCTG GTCCTTTGGG
       TCCGGGCCAC CCCCGACCCC GCGGTACGCG CACGCGCGAC CAGGAAACCC
5851   CGCTAACTGC GTGCGCGCTG GGAATTGGCG CTAATTGCGC GTGCGCGCTG
       GCGATTGACG CACGCGCGAC CCTTAACCGC GATTAACGCG CACGCGCGAC
5901   GGACTCAATG GCGCTAATCG CGCGTGCGTT CTGGGGCCCG GGCGCTTGCG
       CCTGAGTTAC CGCGATTAGC GCGCACGCAA GACCCCGGGC CCGCGAACGC
5951   CCACTTCCTG CCCGAGCCGC TGGCGCCCGA GGGTGTGGCC GCTGCGTGCG
       GGTGAAGGAC GGGCTCGGCG ACCGCGGGCT CCCACACCGG CGACGCACGC
6001   CGCGCGCGAC CCGGTCGCTG TTTGAACCGG GCGGAGGCGG GGCTGGCGCC
       GCGCGCGCTG GGCCAGCGAC AAACTTGGCC CGCCTCCGCC CCGACCGCGG
6051   CGGTTGGGAG GGGGTTGGGG CCTGGCTTCC TGCCGCGCGC CGCGGGGACG
       GCCAACCCTC CCCCAACCCC GGACCGAAGG ACGGCGCGCG GCGCCCCTGC
6101   CCTCCGACCA GTGTTTGCCT TTTATGGTAA TAACGCGGCC GGCCCGGCTT
       GGAGGCTGGT CACAAACGGA AAATACCATT ATTGCGCCGG CCGGGCCGAA
6151   CCTTTGTCCC CAATCTGGGC GCGCGCCGGC GCCCCTGGC GGCCTAAGGA
       GGAAACAGGG GTTAGACCCG CGCGCGGCCG CGGGGGACCG CCGGATTCCT
6201   CTCGGCGCGC CGGAAGTGGC CAGGGCGGGG GCGACTTCGG CTCACAGCGC
       GAGCCGCGCG GCCTTCACCG GTCCCGCCCC CGCTGAAGCC GAGTGTCGCG
6251   GCCCGGCTAT TCTCGCAGCT CACCATGCCG GTCGCCACCA TGATACCGTC
       CGGGCCGATA AGAGCGTCGA GTGGTACGGC CAGCGGTGGT ACTATGGCAG
6301   GAC
       CTG
```

Figure 8a:

CET 600
10316 bp

ACTIN MFI
NotI (778)
NotI (929)
KpnI (2134)

RNP MFI
NotI (4730)

Kan/Neo

SV40 pA
NotI (6985)
BamHI (6964)
KpnI (6953)
SalI (6943)
HindIII (6926)

CMV Promoter
HindIII (6307)

FIGURE 8b:

Kan/Neo

SV40 pA

NotI (6985)

BamHI (6964)

KpnI (6953)

SalI (6943)

HinDIII (6926)

CMV Promoter

HinDIII (10)

NotI (1587)

RNP MFI

CET 601
10316 bp

KpnI (4191)

NotI (5539)

NotI (5388)

ACTIN MFI

EP 1 430 124 B1

## Figure 9
### Comparison of RNP UCOE with RNP/HP-1/actin artificial UCOE
### EGFP Expression in CHO Cells
### Median Fluorescence

Legend:
- 14 Days
- 16 Days
- 21 Days
- 29 Days
- 36 Days
- 44 Days
- 50 Days
- 57 Days
- 64 Days
- 79 Days
- 99 Days
- 106 Days
- 120 Days
- 127 Days

X-axis categories: CMV, CET 220, CET 221, CET 610, CET 611

EP 1 430 124 B1

Figure 10
Comparison of RNP UCOE with RNP/HP-1/actin artificial
UCOE EGFP Expression in CHO Cells
% Positive Cells

EP 1 430 124 B1

human beta-actin promoter

human CMV promoter

HSV TK polyA

SalI (270)

RNP UCOE

BglII (5093)

EcoRI (5445)

pMB1 ori

MfeI (11809)

Amp

PvuI (11343)

ScaI (11231)

MfeI (10784)

lacO3

lacO1

SV40 polyA

neomycin

SV40 ori

mPGK polyA

BamHI (8585)

PacI (8264)

murine CMV promoter

EcoRI (7166)

HindIII (7159)

ClaI (6501)

**pBDUneo100**
12701 bp

Figure IIa :

**pMB1 ori**

**Amp**

**HSV TK polyA**

*Mfe*I (11217)

*Sal*I (270)

*Pvu*I (10751)

*Sca*I (10639)

*Mfe*I (10192)

**human beta-actin promoter**

**lacO3**

**lacO1**

**SV40 polyA**

**pBDUneo200**
**12109 bp**

**neomycin**

**SV40 ori**

**RNP UCOE**

**murine PGK polyA**

*Bam*HI (7993)

*Bgl*II (4501)

*Eco*RI (4853)

*Pac*I (7672)

**murine CMV promoter**

*Eco*RI (6574)

*Cla*I (5909)

*Hin*dIII (6567)

FIGURE 11b:

Figure 11c:

Figure 11 d:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 9902357 W
- WO 0005393 A
- WO 9807876 A

### Non-patent literature cited in the description

- **KAUFMAN.** *Methods Enzymol.,* 1990, vol. 185, 537-566
- **NEEDHAM et al.** *Nucleic Acids Res.,* 1992, vol. 20, 997-1003
- **NEEDHAM et al.** *Protein Expr. Purif.,* 1995, vol. 6, 124-131
- **BIRD et al.** *Cell,* 1985, vol. 40, 91-99
- **BIRD A.P.** *Nature,* 1986, vol. 321, 209-213
- **TAZI ; BIRD.** *Cell,* 1990, vol. 60, 909-920
- **WISE ; PRAVTCHEVA.** *Genomics,* 1999, vol. 60, 258-271
- **KUNDU ; RAO.** *J. Biochem.,* 1999, vol. 125, 217-222
- **SAMBROOK et al.** *Molecular Cloning; A Laboratory Approach,* 1989
- **YOUSSOUFIAN ; LODISH.** Transcriptional inhibition of the murine erythropoietin receptor gene by an upstream repetitive element. *Mol Cell Biol,* 1993, vol. 13 (1), 98-104
- **DAVID S. LATCHMAN.** Eukaryotic Transcription Factors. Academic Press Ltd
- **SAMBROOK et al.** Molecular Cloning: A Laboratory Manual. Cold Spring Harbour Laboratory, Cold Spring Harbour, 1989
- **MARSTON, F.** DNA Cloning Techniques: A Practical Approach. IRL Press, 1987, vol. III
- DNA Cloning. **F M AUSUBEL E.** Current Protocols in Molecular Biology. John Wiley & Sons, Inc, 1994
- **CHOWDHURY et al.** *Science,* 1991, vol. 254, 1802-1805
- **WILSON.** *Hum. Gene Ther.,* 1992, vol. 3, 179-222
- **GORDON et al.** *Proc. Natl. Acad Sci. USA,* 1980, vol. 77, 7380
- **HARBERS et al.** *Nature,* 1981, vol. 293, 540
- **WAGNER et al.** *Proc. Natl. Acad. Sci. USA,* 1981, vol. 78, 5016
- **WAGNER et al.** *Proc. Natl. Acad. Sci. USA,* 1981, vol. 78, 6376
- **HAMMER et al.** *Nature,* 1985, vol. 315, 680
- **G. HICKS et al.** *Nature Genetics,* vol. 16, 338-334
- **DILLON, N. ; GROSVELD, F.** Chromatin domains as potential units of eukaryotic gene function. *Curr. Opin. Genet. Dev.,* 1994, vol. 4, 260-264
- **HIGGS, D.R.** Do LCRs open chromatin domains?. *Cell,* 1998, vol. 95, 299-302
- **PALMITER, R.D. ; BRINSTER, R.L.** Germline transformation of mice. *Ann. Ref Genet.,* 1986, vol. 20, 465-499
- **ALLEN, N.D. et al.** Transgenes as probes for active chromosomal domains in mouse development. *Nature,* 1988, vol. 333, 852-855
- **BONNEROT, C. ; GRIMBER, G. ; BRIAND, P. ; NICOLAS, J.F.** Patterns of expression of position-dependent integrated transgenes in mouse embryo. *Proc. Natl. Acad Sci. USA,* 1990, vol. 87, 6331-6335
- **KIOUSSIS, D. ; FESTENSTEIN, R.** Locus control regions: overcoming heterochromatin-induced gene inactivation in mammals. *Curr. Opin. Genet. Dev.,* 1997, vol. 7, 614-619
- **PIKAART, , M.J. ; RECILLAS-TARGA, F. ; FELSENFELD, G.** Loss of transcriptional activity of a transgene is accompanied by RNA methylation and histone deacetylation and is prevented by insulators. *Genes Dev.,* 1998, vol. 12, 2852-2862
- **FUSSENEGGER, M. ; BAILEY, J.E. ; HAUSER, H. ; MUELLER, P.P.** Genetic optimization of recombinant glycoprotein production by mammalian cells. *Trends Biotech.,* 1999, vol. 17, 35-42
- **FRASER, P. ; GROSVELD, F.** Locus control regions; chromatin activation and transcription. *Curr. Opin. Cell Biol.,* 1998, vol. 10, 361-365
- **LI, Q. ; HARJU, S. ; PETERSON, K.R.** Locus Control Regions: coming of age at a decade plus. *Trends Genet.,* 1999, vol. 15, 403-408
- **BULGER, M. ; GROUDINE, M.** Looping versus linking: toward a model for long-distance gene activation. *Genes Dev.,* 1999, vol. 13, 2465-2477
- **GROSVELD, F.** Activation by locus control regions?. *Curr. Opin. Genet. Dev.,* 1999, vol. 9, 152-157

- **BENDER, M.A. ; BULGER, M. ; CLOSE, J. ; GROU-DINE, M.** ß-globin Gene Switching and Dnase I Sensitivity of the Endogenous ß-globin Locus in Mice Do Not Require the Locus Control Region. *Mol. Cell,* 2000, vol. 5, 387-393
- **ORTIZ, B.D. ; CADO, D. ; CHEN, V. ; DIAZ, P.W. ; WINOTO, A.** Adjacent DNA elements dominantly restrict the ubiquitous activity of a novel chromatin-opening region to specific tissues. *EMBO J.,* 1997, vol. 16, 5037-5045
- **ORTIZ, B.D. ; CADO, D. ; WINOTO, A.** A new element within the T-cell receptor alpha locus required for tissue-specific locus control region activity. *Mol. Cell. Biol.,* 1999, vol. 19, 1901-1909
- **ANTEQUERA, F. ; BIRD, A.** Number of CpG islands and genes in human and mouse. *Proc. Natl. Acad Sci. USA,* 1993, vol. 90, 1195-11999
- **CROSS, S.H. ; BIRD, A.P.** CpG islands and genes. *Curr. Opin, Genet. Dev.,* 1995, vol. 5, 309-314
- **TAZI, J. ; BIRD, A.** Alternative chromatin structure at CpG islands. *Cell,* 1990, vol. 60, 909-920
- **IMBERT, G. ; TROTTIER, Y. ; BECHMANN, J. ; MANDEL, J.L.** The gene for the TATA binding protein (TBP) that contains a highly polymorphic protein coding CAG repeat maps to 6q27. *Genomics,* 1994, vol. 21, 667-668
- **PURRELLO, M. et al.** Physical mapping at 6q27 of the locus for the TATA-box binding protein, the DNA binding subunit of TFIID and a component of SL1 and TFIIIB, strongly suggests that it is single copy in the human genome. *Genomics,* 1994, vol. 22, 94-100
- **TRACHTULEC, Z et al.** Linkage of TATA-binding protein and proteasome subunit C5 genes in mice and humans reveals synteny conserved between mammals and invertebrates. *Genomics,* 1997, vol. 44, 1-7
- **OWENS, G.P. ; HAHAN, W.E. ; COHEN, J.J.** Identification of mRNAs associated with programmed cell death in immature thymocytes. *Mol. Cell. Biol.,* 1991, vol. 11, 4177-4188
- **CHALUT, C. ; GALLOIS, Y. ; POTERSZMAN, A. ; MONCOLLIN, V. ; EGLY, J.-M.** Genomic structure of the human TATA-box-binding protein (TBP. *Gene,* 1995, vol. 161, 277-282
- **SCHMIDT, E.E. ; SCHIBLER, U.** High accumulation of components of the RNA polymerase II transcription machinery in rodent spermatids. *Development,* 1995, vol. 121, 2373-2383
- **BIAMONTI, G. ; RUGGIU, M. ; SACCONE, S. ; DELLA VALLE, G. ; RIVA, S.** Two homologous genes, originated by duplication, encode the human hnRNP proteins A2 and A1. *Nucl. Acids Res.,* 1994, vol. 22, 1996-2002
- **KOZU, T. ; HENRICH, B. ; SCHAFER, K.P.** Structure and expression of the gene (HNRPA2B1) encoding the human hnRNP protein A2/B1. *Genomics,* 1995, vol. 25, 365-371
- **YE, Q. ; WORMAN, H.J.** Interaction between an integral protein of the nuclear envelope inner membrane and human chromodomain proteins homologous to Drosophila HP1. *J. Biol. Chem.,* 1996, vol. 271, 14653-14656
- **JAMES, T.C. ; ELGIN, S.C.R.** Identification of a nonhistone chromosomal protein associated with heterochromatin in Drosophila melanogaster and its gene. *Mol. Cell. Biol.,* 1986, vol. 6, 3861-3872
- **SINGH, P.B. et al.** A sequence motif found in a drosophila heterochromatin protein is conserved in animals and plants. *Nucl. Acids Res.,* 1991, vol. 19, 789-794
- **GILLIAND, G. ; PERRIN,M S. ; BLANCHARD, K. ; BUNN, H.F.** Analysis of cytokine Mrna and DNA: detection and quantitation by competitive polymerase chain reaction. *Proc. Natl. Acad. Sci. USA,* 1990, vol. 87, 2725-2729
- **FURTH, P.A. ; HENNIGHAUSEN, L. ; BAKER, C. ; BEATTY, B. ; WOYCHICK, R.** The viarability in activity of the universally expressed human cytomegalovirus immediate early gene 1 promoter/enhancer in transgenic mice. *Nucl. Acids Res.,* 1991, vol. 19, 6205-6208
- **RAY, P. et al.** Ectopic expression of a c-kitW42 minigene in transgenic mice: recapitulation of W phenotypes and evidence for c-kit function in melanoblast progenitors. *Genes Dev.,* 1991, vol. 5, 2265-2273
- **YAMASHITA, T. et al.** High level expression of human $\alpha$-fetoprotein in transgenic mice. *Biochem, Biophys. Res. Comm.,* 1993, vol. 191, 715-720
- **MILOT, E. et al.** Heterochromatin effects on the frequency and duration of LCR-mediated gene transcription. *Cell,* 1996, vol. 87, 104-114
- **FESTENSTEIN, R. et al.** Locus control region function and heterochromatin-induced position effect variegation. *Science,* 1996, vol. 271, 1123-1125
- **SABBATTINI, P ; GEORGIOU, A. ; SINCLAIRE, C. ; DILLON, N.** Analysis of mice with single and multiple copies of transgenes reveals a novel arrangement for the λ5-V-preBI locus control region. *Mol. Cell. Biol.,* 1999, vol. 19, 671-679
- **NG, S.Y. et al.** Evolution of the functional human ß-actin gene and its multi-pseudogene family: conservation of noncoding regions and chromosomal dispersion of pseudogenes. *Mol. Cell. Biol.,* 1985, vol. 5, 2720-2732
- **PRAVTCHEVA, D.D. ; WISE, T.L. ; ENSOR, N.J. ; RUDDLE, F.H.** Mosaic expression of an HPRT transgene integrated in a region of Y heterochromatin. *J. Exp. Zool.,* 1994, vol. 268, 452-468
- **BELL, A.C. ; FELSENFIELD, G.** Stopped at the border: boundaries and insulators. *Curr. Opin. Genet. Dev.,* 1999, vol. 9, 191-198

- **WINSTON, J.H. ; HONG, L. ; DATTA, S.K. ; KELLEMS, R.E.** An intron 1 regulatory region from the murine adenosine deaminase gene can activate heterologous promoters for ubiquitous expression in transgenic mice. *Somat. Cell Mol. Genet.,* 1996, vol. 22, 261-278
- **HART, C.M. ; LAEMMLI, U.K.** Facilitation of chromatin dynamics by SARs. *Curr. Opin. Genet. Develop.,* 1998, vol. 8, 519-525
- **KLEHR, D. ; MAASS, K. ; BODE, J.** Scaffold-attached regions from the human interferon ß domain can be used to enhance the stable expression of genes under the control of various promoters. *Biochemistry,* 1991, vol. 30, 1264-1270
- **MCKNIGHT, R.A. ; SHAMAY, L. ; SANKARAN, L. ; WALL, R.J. ; HENNINGHAUSEN, L.** Matrix-attachment regions can impart position-independent regulation of a tissue-specific gene in transgenic mice. *Proc. Natl. Acad Sci. USA,* 1992, vol. 89, 6943-6947
- **VAN DRUNEN, C.M. et al.** A bipartite sequence element associated with matrix/scaffold attachment regions. *Nucl. Acids Res.,* 1999, vol. 27, 2924-293 0
- **VERMA, I.M. ; SOMIA, N.** Gene Therapy - promises, problems and prospects. *Nature,* 1997, vol. 389, 239-242
- **BROWN, S.A. ; KINGSTON, R.E.** Disruption of downstream chromatin directed by a transcriptional activator. *Genes Dev.,* 1997, vol. 11, 3 116-3121
- **ORPHANIDES, G. ; LEROY, G. ; CHANG, C.H. ; LUSE, D.S. ; REINBERG, D.** FACT, a factor that facilitates transcript elongation through nucleosomes. *Cell,* 1998, vol. 92, 105-116
- **SCHNITZLER, G. ; SIF, S. ; KINGSTON, R.E.** Human SWI/SNF interconverts a nucleosome between its base state and a stable remodelled state. *Cell,* 1998, vol. 94, 17-27

- **TRAVERS, A.** Chromatin modification by DNA tracking. *Proc. Natl. Acad. Sci. USA,* 1999, vol. 96, 13634-13637
- **ASHE, H.L. ; MONKS, J. ; WIJGERDE, M. ; FRASER, P. ; PROUDFOOT, N.J.** Intergenic transcription and transinduction of the human ß-globin locus. *Genes Dev,* 1997, vol. 11, 2494-2509
- **ROGAN, D.F. ; COUSINS, D.J. ; STAYNOV, D.Z.** Intergenic transcription occurs throughout the human IL-4/IL-13 gene cluster. *Biochem, Biophys, Res. Commun.,* 1999, vol. 255, 556-561
- **GRIBNAU, J. ; DIDERICH, K. ; PRUZINA, S. ; CALZORLARI, R. ; FRASER P.** Intergenic Transcription and Developmental Remodeling of Chromatin Subdomains in the Human ß-globin Locus. *Mol. Cell,* 2000, vol. 5, 377-386
- **VYAS, P. et al.** Cis-acting sequences regulating expression of the human α-globin cluster lie within constitutively open chromatin. *Cell,* 1992, vol. 69, 781-793
- **IOANNOU, P.A. et al.** A new bacteriophage P1-derrived vector for the propagation of large human DNA fragments. *Nature Gene,* 1994, vol. 6, 84-89
- **RAGUZ, S. et al.** Muscle-specific locus control region activity associated with the human desmin gene. *Dev. Biol.,* 1998, vol. 201, 26-42
- Transcriptional analysis using transgenic animals. **DILLON, N. ; GROSVELD, F.** Gene Transcription: A practical approach. IRL Press, 1993, 153-188
- **MORGENSTERN, J.P.** Advanced mammalian gene transfer: high titre retroviral vectors with multiple drug selection markers and a complementary helper-free packaging cell line. *Nucl. Acids Res,* 1990, vol. 18, 3587-3595
- **HORZ, W. ; ALTENBURGER, W.** Nucleotide sequence of mouse satellite DNA. *Nucl. Acids Res.,* 1981, vol. 9, 683-696
- **MONFOUILLOUX, S. et al.** Recent human-specific spreading of a subtelomeric domain. *Genomics,* 1998, vol. 51, 165-176